(19) Europäisches Patentamt / European Patent Office / Office européen des brevets

(11) EP 2 342 185 B1

(12) **EUROPÄISCHE PATENTSCHRIFT**

(45) Veröffentlichungstag und Bekanntmachung des Hinweises auf die Patenterteilung:
**13.01.2016 Patentblatt 2016/02**

(21) Anmeldenummer: **09778863.2**

(22) Anmeldetag: **08.10.2009**

(51) Int Cl.:
***C07D 239/47*** *(2006.01)* ***A61K 31/505*** *(2006.01)*
***A61P 35/00*** *(2006.01)*

(86) Internationale Anmeldenummer:
**PCT/EP2009/007213**

(87) Internationale Veröffentlichungsnummer:
**WO 2010/046034 (29.04.2010 Gazette 2010/17)**

(54) **SULFONSUBSTITUIERTE ANLINOPYRIMIDINDERIVATIVE ALS CDK-INHIBITOREN, DEREN HERSTELLUNG UND VERWENDUNG ALS ARZNEIMITTEL**

SULFON SUBSTITUTED ANILINE PYRIMIDINE DERIVATIVES AS CDK INHIBITORS, THEIR MANUFACTURE AND USE AS MEDICINE

DÉRIVÉS D'ANILINO-PYRIMIDINE SUBSTITUÉS PAR SULFONE EN TANT QU'INHIBITEURS DE CDK, LEUR FABRICATION ET LEUR UTILISATION EN TANT QUE MÉDICAMENTS

(84) Benannte Vertragsstaaten:
**AT BE BG CH CY CZ DE DK EE ES FI FR GB GR HR HU IE IS IT LI LT LU LV MC MK MT NL NO PL PT RO SE SI SK SM TR**

(30) Priorität: **21.10.2008 EP 08167115**

(43) Veröffentlichungstag der Anmeldung:
**13.07.2011 Patentblatt 2011/28**

(73) Patentinhaber: **Bayer Intellectual Property GmbH**
**40789 Monheim (DE)**

(72) Erfinder:
- **LÜCKING, Ulrich**
  **10317 Berlin (DE)**
- **SIEMEISTER, Gerhard**
  **13503 Berlin (DE)**
- **LIENAU, Philip**
  **10997 Berlin (DE)**
- **JAUTELAT, Rolf**
  **42781 Haan (DE)**
- **SCHULZE, Julia**
  **10407 Berlin (DE)**

(74) Vertreter: **BIP Patents**
**c/o Bayer Intellectual Property GmbH**
**Alfred-Nobel-Straße 10**
**40789 Monheim am Rhein (DE)**

(56) Entgegenhaltungen:
**WO-A-02/096887**
**WO-A-03/032997**
**WO-A-2005/037800**
**WO-A-2008/107096**

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

EP 2 342 185 B1

## Beschreibung

Die vorliegende Erfindung betrifft sulfonsubsituierte Anilino-Pyrimidinderivate, deren Verfahren zur Herstellung, sowie deren Verwendung als Medikament zur Behandlung verschiedener Erkrankungen.

Die Zyklin-abhängigen Kinasen (cyclin-dependent kinase, CDK) sind eine Enzymfamilie, die eine wichtige Rolle bei der Regulation des Zellzyklusses spielt und somit ein besonders interessantes Ziel für die Entwicklung kleiner inhibitorischer Moleküle darstellt. Selektive Inhibitoren der CDKs können zur Behandlung von Krebs oder anderen Erkrankungen, die Störungen der Zellproliferation zur Ursache haben, verwendet werden.

Pyrimidine und Analoga sind bereits als Wirkstoffe beschrieben wie beispielsweise die 2-Anilino-Pyrimidine als Fungizide (DE 4029650) oder substituierte Pyrimidinderivate zur Behandlung von neurologischen oder neurodegenerativen Erkrankungen (WO 99/19305). Als CDK-Inhibitoren werden unterschiedlichste Pyrimidinderivate beschrieben, beispielsweise 2-Amino-4-substituierte Pyrimidine (WO 01/ 14375), Purine (WO 99/02162), 5-Cyano-Pyrimidine (WO 02/04429), Anilinopyrimidine (WO 00/12486) und 2-Hydroxy-3-N,N-dimethylaminopropoxy-Pyrimidine (WO 00/39101).

Insbesondere wurden in WO 02/096888 und WO 03/076437 Pyrimidinderivate offenbart, die inhibitorische Wirkungen bezüglich CDKs aufweisen.

WO 2005/037800 offenbart offene sulfoximinsubsituierte Anilino-Pyrimidinderivate als Inhibitoren der Zyklin-abhängigen Kinasen. Beispielhaft belegt sind Strukturen, die in der 5-Position des Pyrimidins entweder nicht oder mit Halogen, insbesondere mit Brom substituiert sind. Einen 5-Trifluormethylsubstituenten weist keine der spezifisch offenbarten Strukturen auf.

WO 2003/032997 offenbart sulfonsubstituierte Anilinopyrimidine, für die allerdings in der Position 4 des Pyrimidins obligat ein stickstoffhaltiger Rest vorgesehen ist. Die den erfindungsgemäßen Strukturen am nächsten kommende spezifisch offenbarte Struktur ist die Struktur 692 des Beispiels 1.

Ausgehend von diesem Stand der Technik besteht die Aufgabe der vorliegenden Erfindung Verbindungen bereitzustellen, die die Aktivität der Zyklin-abhängigen Kinasen stärker inhibieren als die Verbindungen des Standes der Technik. Weiterhin sollen die Verbindungen selektiver gegenüber der Inhibition der VEGF-Rezeptorkinase-2 (VEGF-R2) sein. Die Verbindungen sollen gut permeabel in absorptiver Richtung und gering permeabel in Efflux-Richtung sein. Insbesondere sollen die Verbindungen auch in Chemotherapie-resistenten Tumorzellen stark antiproliferativ wirken.

Es wurden nun Verbindungen der allgemeinen Formel (Ia) gefunden

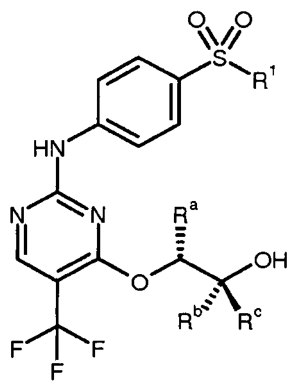

(Ia),

in der

| | |
|---|---|
| $R^1$ | für eine Methylgruppe oder für einen Cyclopropylring steht, und |
| $R^a$ und $R^b$ | für eine Methylgruppe stehen, und |
| $R^c$ | für Wasserstoff oder eine Methylgruppe steht, |

sowie deren Salze, Diatereomere und Enantiomere.

$R^1$ steht für eine Methylgruppe oder für einen Cyclopropylring.

$R^a$ und $R^b$ stehen für eine Methylgruppe und $R^c$ steht für Wasserstoff oder eine Methylgruppe.

Formel (Ia) fasst diese Gruppe von Verbindungen zusammen.

Bevorzugt sind folgende Verbindungen

- (2R,3R)-3-[2-(4-Cyclopropansulfonyl-phenylamino)-5-trifluormethyl-pyrimidin-4-yloxy]-butan-2-ol

- (2R,3R)-3-[2-(4-Methansulfonyl-phenylamino)-5-trifluormethyl-pyrimidin-4-yloxy]-butan-2-ol

- (R)-3-[2-(4-Methansulfonyl-phenylamino)-5-trifluormethyl-pyrimidin-4-yloxy]-2-methyl-butan-2-ol

Die erfindungsgemäßen Verbindungen eignen sich zur Behandlung

- von Krebs, wie solide Tumore, Tumormetastasen, und Hämatologische Tumoren, insbesondere:

  Kopf- und Halstumore; Lungen- und Bronchialtumore; Gastrointestinaltumore wie z.B. Magenkarzinom, Kolorektales Karzinom, Pankreaskarzinom, Hepatozelluläres Karzinom; Endokin aktive Tumore; Mammakarzinome und Gynäkologische Tumore; Urogenitaltumore, wie z.B. Nierenzellkarzinom, Harnblasenkarzinom, Prostatakarzinom; Tumore der Haut; Sarkome; Leukämien und Lymphome.
  • von viralen Erkrankungen, sowie
  • von kardiovaskulären Erkrankungen wie Stenosen, Arteriosklerosen und Restenosen, Stentinduzierte Restenosen.

Die Formulierung der erfindungsgemäßen Verbindungen zu pharmazeutischen Präparaten erfolgt in an sich bekannter Weise, indem man den oder die Wirkstoffe mit den in der Galenik gebräuchlichen Hilfsstoffen in die gewünschte Applikationsform überführt.
Als Hilfsstoffe können dabei beispielsweise Trägersubstanzen, Füllstoffe, Sprengmittel, Bindemittel, Feuchthaltemittel, Gleitmittel, Ab- und Adsorptionsmittel, Verdünnungsmittel, Lösungsmittel, Cosolventien, Emulgatoren, Lösungsvermittler, Geschmackskorrigentien, Färbemittel, Konservierungs-, Stabilisierungs-, Netzmittel, Salze zur Veränderung des osmotischen Drucks oder Puffer zum Einsatz kommen.
Dabei ist auf Remington's Pharmaceutical Science, 15th ed. Mack Publishing Company, East Pennsylvania (1980) hinzuweisen.
Die pharmazeutischen Formulierungen können
in <u>fester</u> Form, zum Beispiel als Tabletten, Dragees, Pillen, Suppositorien, Kapseln, transdermale Systeme oder
in <u>halbfester</u> Form, zum Beispiel als Salben, Cremes, Gele, Suppositiorien, Emulsionen oder in <u>flüssiger</u> Form, zum Beispiel als Lösungen, Tinkturen, Suspensionen oder Emulsionen vorliegen.
Hilfsstoffe im Sinne der Erfindung können beispielsweise Salze, Saccharide (Mono-, Di-, Tri-, Oligo-, und/oder Polysaccharide), Proteine, Aminosäuren, Peptide, Fette, Wachse, Öle, Kohlenwasserstoffe sowie deren Derivate sein, wobei die Hilfsstoffe natürlichen Ursprungs sein können oder synthetisch bzw. partial synthetisch gewonnen werden können.
Für die orale oder perorale Applikation kommen insbesondere Tabletten, Dragees, Kapseln, Pillen, Pulver, Granulate, Pastillen, Suspensionen, Emulsionen oder Lösungen in Frage.
Für die parenterale Applikation kommen insbesondere Suspensionen, Emulsionen und vor allem Lösungen in Frage.

<u>**Herstellung der erfindungsgemäßen Verbindungen**</u>

Die nachfolgenden Beispiele erläutern die Herstellung der erfindungsgemäßen Verbindungen, ohne den Umfang der beanspruchten Verbindungen auf diese Beispiele zu beschränken.
Die erfindungsgemäßen Verbindungen können hergestellt werden durch ein Verfahren, das durch folgende Schritte gekennzeichnet ist:

$a_1$) Funktionalisierung der 4-Position von 2,4-Dichlor-5-jod-pyrimidin (1) durch Umsetzung mit einem Alkohol der Formel (2) unter Bildung eines Intermediates der Formel (3)

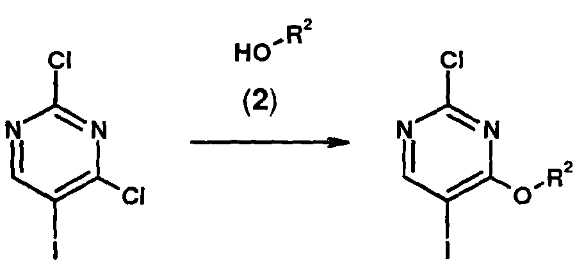

(1) (3)

und nachfolgende Umsetzung von Intermediat (3) unter Bildung des 5-$CF_3$ Intermediates (4)

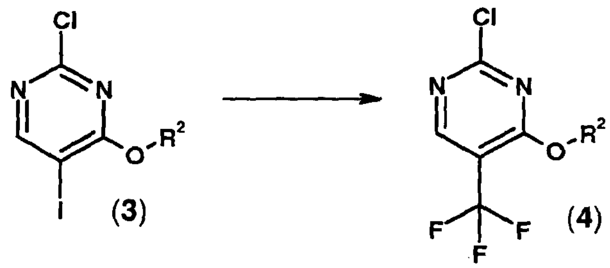

(3) (4)

oder alternativ

$a_2$) direkte Umsetzung von 2,4-Dichlor-5-trifluormethyl-pyrimidin (5) und einem Alkohol der Formel (2) unter Bildung des 5-$CF_3$ Intermediates (4).

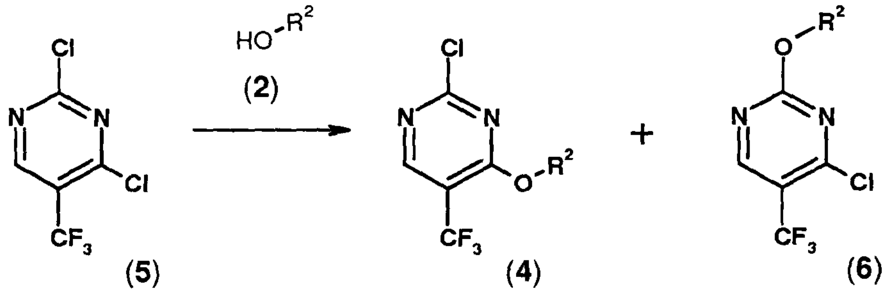

(2)

(5) (4) + (6)

b) Oxidation eines Thioethers der Formel **(7)** zum Sulfon der Formel (**8**).

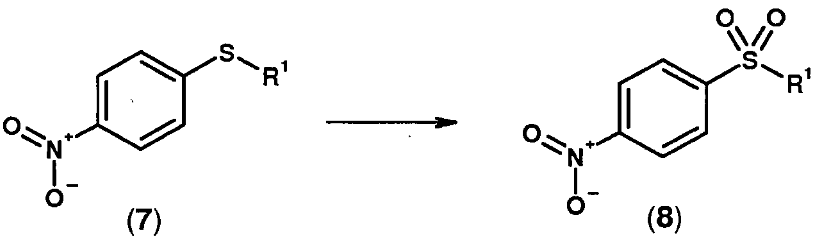

(7) (8)

c) Reduktion der Verbindung der Formel **(8)** zu einer Verbindung der Formel **(9).**

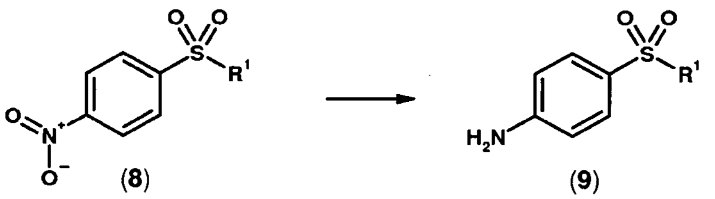

(**8**) (9)

d) Kupplung der Verbindungen der Formel **(4)** und **(9).**

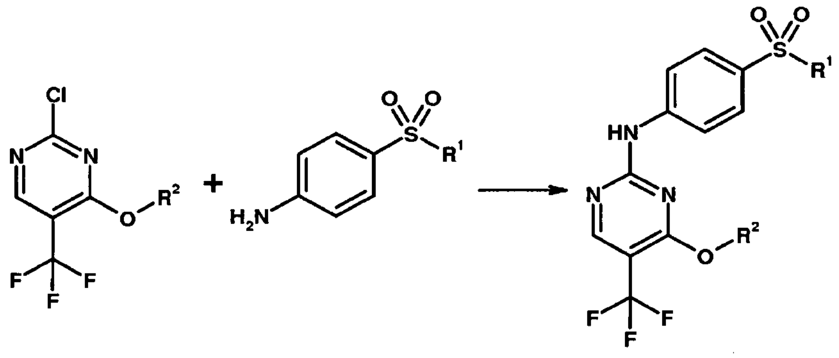

(4) (9) (I)

Gegebenfalls erfordert die Herstellung der erfindungsgemäßen Verbindungen das Einführen und anschließende Abspalten von Schutzgruppen (siehe z.B.: P.J. Kocienski, Protecting Groups, Georg Thieme Verlag Stuttgart, New York, 1994) wie z.B. in der Seitenkette der 4-Position.

Verfahrensschritt $a_1$)

Die Umsetzung von 2,4-Dichlor-5-jod-pyrimidin **(1)** mit einem Alkohol der Formel **(2)** unter basischen Bedingungen ermöglicht die Darstellung eines Produktes der Formel **(3)** (siehe z.B.: (a) U. Lücking et al, WO 2007/071455). Besonders geeignet für die Darstellung ist die beschriebene Verwendung von Natriumhydrid.

Für den Austausch eines Halogens gegen eine Trifluormethylgruppe in einem stickstoffhaltigen Heteroaromaten stehen prinzipiell verschiedene Methoden zur Verfügung (siehe z.B: a) G.E. Carr, R.D. Chambers, T.F. Holmes, J. Chem. Soc. Perkin Trans. 1, 1988,921; b) F. Cottet, M. Schlosser, Eur. J. Org. Chem. 2002, 327; c) F.G. Njoroge et al, J. Med. Chem 1997, 40, 4290).

Besonders geeignet für den Austausch des Jods in der 5-Position des Pyrimidins **(3)** gegen eine $CF_3$-Gruppe unter Bildung einer Verbindung der Formel **(4)** ist die beschriebene Verwendung Kupfer(I)jodid, Kaliumfluorid und (Trifluormethyl)-trimethylsilan in N-Methyl-2-pyrrolidinon und THF.

Verfahrensschritt $a_2$)

Die Umsetzung von 2,4-Dichlor-5-trifluormethyl-pyrimidin (5) mit einem Alkohol der Formel (2) unter basischen Bedingungen ermöglicht die Darstellung der Produkte **(4)** und **(6).** Die Regioisomeren können in der Regel chromatographisch getrennt werden. (siehe z.B.: (a) T.M. Caldwell et al, WO 2006/081388, S. 50, Example 1, D). Besonders geeignet für die Darstellung ist die beschriebene Verwendung von Natriumhydrid.

Verfahrensschritt b)

Eine Verbindung der Formel **(7)** wird zum Sulfon der Formel **(8)** oxidiert. Für die Überführung eines Thioethers in ein Sulfon stehen zahlreiche Methoden zur Verfügung, z.B. unter Verwendung der Oxidationsmittel Wasserstoffperoxid oder Kaliumpermanganat. Besonders geeignet für die Darstellung von Verbindungen der Formel **(8)** ist die beschriebene Verwendung von meta-Chlorperbenzoesäure (MCPBA).

Verfahrensschritt c)

Für die anschließende Reduktion der aromatischen Nitrogruppe zu einer Verbindung der Formel **(9)** stehen prinzipiell eine Reihe von Reaktionsbedingungen zur Verfügung (siehe z.B.: R.C. Larock, Comprehensive Organic Transformations, VCH, New York, 1989, 411). Besonders geeignet ist beispielsweise die beschriebene Hydrierung unter Verwendung von Raney Nickel in THF.

Verfahrensschritt d)

Eine Verbindung der Formel **(4)** kann mit einem Anilin der Formel **(9)** zu einer Verbindung der Formel **(I)** umgesetzt werden (siehe z.B.: (a) J. Bryant et al, WO 2004/048343).

## Allgemeine Hinweise

Alle Reaktionen mit oxidations- oder hydrolyseempfindlichen Verbindungen wurden unter Argon und mit getrockneten Lösungsmitteln durchgeführt.

Die Substanzbenennung erfolgte unter Verwendung des Programms *Autonom 2000 Name,* welches in MDL ISIS Draw implementiert ist.

**Abkürzungen**

| Abkürzung | Bedeutung |
|---|---|
| Ac | Acetyl |
| Aloc | Allyloxycarbonyl |
| Boc | tert-Bytyloxycarbonyl |
| BOM | Benzyloxymethyl |
| br | Broad |
| CI | Chemical ionisation |
| d | Dublett |
| dd | Dublett vom Dublett |
| DCM | Dichlormethan |
| DMF | *N,N*-Dimethylformamid |
| DMSO | Dimethylsulfoxid |
| ESI | Electrospray ionisation |
| HPLC | High performance liquid chromatopraphy |
| m | Multiplett |
| MEM | (2-Methoxyethoxy)methyl |
| MOM | Methoxymethyl |
| MS | Mass spectrometry |
| MTM | Methylthiomethyl |
| NMP | N-Methyl-2-pyrrolidinon |
| NMR | Nuclear magnetic resonance spectroscopy: Chemische Verschiebung ($\delta$) wird in ppm angegeben. |
| Pg | Protecting group umfassend Gruppen wie z.B. TMS, TES, TBDMS, TBDPS, TIPS, Benzyl, PMB, Trityl, Allyl, Aloc, MOM, MTM, MEM, BOM, SEM, THP. |
| PMB | p-Methoxybenzyl |
| q | Quartett |
| s | Singulett |
| SEM | $\beta$-(Trimethylsilyl)ethoxymethyl |
| TBDMS | Tert.-Butylsilyldimethyl |
| TBDPS | Tert.-Butylsilyldiphenyl |
| TEA | Triethylamin |
| TES | Triethylsilyl |
| THF | Tetrahydrofuran |
| THP | Tetrahydropyranyl |
| TIPS | Triisopropyl |

(fortgesetzt)

| Abkürzung | Bedeutung |
|---|---|
| TMS | Trimethylsilyl |
| tr | Triplett |

**Beispiel** 1

**(2R,3R)-3-[2-(4-Cyclopropansulfonyl-phenylamino)-5-trifluormethyl-pyrimidin-4-yloxy]-butan-2-ol**

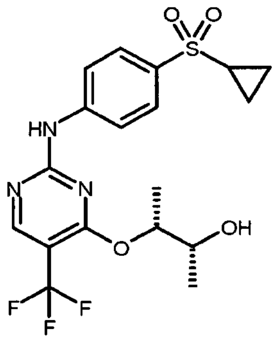

**1a) Herstellung der Zwischenprodukte**

**Verbindung 1.1**

**1-Cyclopropylsulfanyl-4-nitro-benzol**

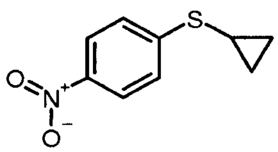

Eine 4%ige Lösung von 3,00 g (40,5 mmol) Cyclopropanthiol (Herstellung nach: E. Block et al, J. Am. Chem. Soc. 1992, 114, 3492) in THF / Diethylether (1:1) wurde portionsweise mit 1,78 g (44,6 mmol) Natriumhydrid (60%) versetzt und 30 Minuten bei Raumtemperatur gerührt. Anschließend erfolgte die portionsweise Zugabe von 6,00 g (38,7 mmol) 1-Fluor-4-nitro-benzol. Der Ansatz wurde 2 Stunden bei 40°C gerührt. Nach dem Abkühlen wurde der Ansatz in Wasser gegeben und mit Benzol extrahiert (3x). Die vereinten organischen Phasen wurden eingeengt und der Rückstand chromatographisch (Hexan / Essigester 95:5) gereinigt. Man erhielt 4,6 g (23,6 mmol; Ausbeute: 61 %) des Produktes. $^{1}$H-NMR (400 MHz, DMSO): δ= 8.12 (m, 2H), 7.54 (m, 2H), 2.35 (m, 1H), 1.16 (m, 2H), 0.61 (m, 2H).

**Verbindung 1.2**

**1-Cyclopropansulfonyl-4-nitro-benzol**

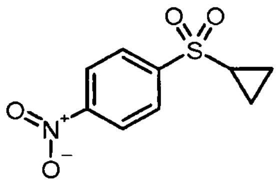

Eine Lösung von 1,00 g (5,12 mmol) 1-Cyclopropylsulfanyl-4-nitro-benzol in 120 ml DCM wurde bei 0°C mit 2,3 g meta-Chlorperbenzoesäure (max. 77%) versetzt und anschließend 4,5 Stunden bei Raumtemperatur gerührt. Der Ansatz wurde unter Rühren in eine gesättigte Natriumhydrogencarbonat-Lösung gegeben. Die organische Phase wurde über einem Whatman Filter filtriert und eingeengt. Der erhaltene Rückstand wurde chromatographisch (DCM / MeOH 95:5) gereinigt. Man erhielt 1,07 g (4,70 mmol; Ausbeute: 92%) des Produktes.
$^1$H-NMR (400 MHz, DMSO): $\delta$ = 8.41 (m, 2H), 8.15 (m, 2H), 2.98 (m, 1H), 1.11 (m, 4h).

**Verbindung 1.3**

**4-Cyclopropansulfonyl-phenylamin**

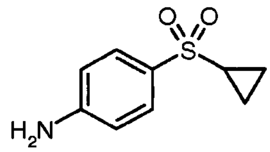

Eine Lösung von 1,60 g (7,0 mmol) 1-Cyclopropansulfonyl-4-nitro-benzol in 50 ml Ethanol und 50 ml THF wurde mit 3,2 g Raney-Nickel (50% wasserfeucht) versetzt und 1,5 Stunden unter Normaldruck bei 0°C hydriert. Der Ansatz wurde filtriert und eingeengt. Man erhielt 1,28 g (6,5 mmol; Ausbeute: 92 %) des Produktes.
$^1$H-NMR (400 MHz, DMSO): $\delta$ = 7.41 (m, 2H), 6.60 (m, 2H), 6.05 (br, 2H), 2.58 (m, 1H), 0.92 (m, 4H).
MS: 198 (ESI+).

**Verbindung 1.4**

**(2R,3R)-3-Benzyloxy-butan-2-ol**

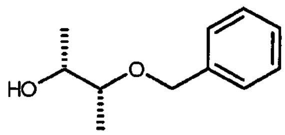

Eine Lösung von 4,0 g (44,4 mmol) (2R,3R)-Butan-2,3-diol in 300 ml THF wurde bei Raumtemperatur mit 5,0 g (44,6 mmol) Kalium-tert.-butylat versetzt und der Ansatz 15 Minuten refluxiert. Der Ansatz wurde auf ca. 50°C abgekühlt und mit 5,3 ml (44,6 mmol) Benzylbromid versetzt. Man refluxierte für 3 Stunden, danach wurde über Nacht bei Raumtemperatur gerührt. Der Ansatz wurde mit Essigester und Natriumchlorid-Lösung verdünnt und anschließend mit 1N Chlorwasserstoff-Lösung (1x) und Natriumchlorid-Lösung (2x) gewaschen. Die organische Phase wurde getrocknet ($Na_2SO_4$), filtriert und eingeengt. Der erhaltene Rückstand wurde chromatographisch (Hexan / Essigester 1:1) gereinigt. Man erhielt 3,4 g (18,9 mmol; Ausbeute: 43 %) des Produktes.
$^1$H-NMR (400 MHz, DMSO): $\delta$ = 7.35 (m, 4H), 7.28 (m, 1H), 4.52 (m, 3H), 3.67 (m, 1H), 3.37 (m, 1H), 1.05 (d, 3H), 1.01 (d, 3H).

**Verbindung 1.5**

**4-((1R,2R)-2-Benzyloxy-1-methyl-propoxy)-2-chlor-5-jod-pyrimidin**

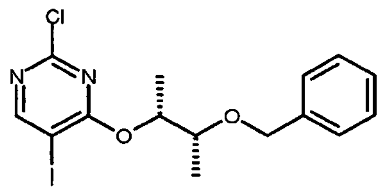

8,55 g (47,4 mmol) (2R,3R)-3-Benzyloxy-butan-2-ol in 56 ml Diethylether wurden bei 0°C unter Rühren portionsweise mit 2,07 g Natriumhydrid (55%) versetzt. Nach 10 Minuten wurde das Eisbad entfernt und weitere 3 Minuten bei Raumtemperatur gerührt. Die gebildete Suspension wurde bei 0°C zu einer Lösung von 6,52 g (23,7 mmol) 2,4-Dichlor-5-jod-pyrimidin in 65 ml Acetonitril gegeben. Der Ansatz wurde 4 Stunden bei 40°C gerührt und anschließend mit verdünnter Natriumchlorid-Lösung versetzt. Man extrahierte mit Essigester (2x). Die vereinten organischen Phasen wurden getrocknet ($Na_2SO_4$), filtriert und eingeengt. Der erhaltene Rückstand wurde chromatographisch (Hexan / Essigester 4:1) gereinigt. Man erhielt 4,12 g (9,8 mmol; Ausbeute: 41%) des Produktes.
$^1$H-NMR (400 MHz, DMSO): $\delta$ = 8.78 (s, 1H), 7.29 (m, 5H), 5.27 (m, 1H), 4.64 (d, 1H), 4.53 (d, 1H), 3.73 (m, 1H), 1.30 (d, 3H), 1.19 (d, 3H).

**Verbindung 1.6**

**4-((1R,2R)-2-Benzyloxy-1-methyl-propoxy)-2-chlor-5-trifluormethyl-pyrimidin**

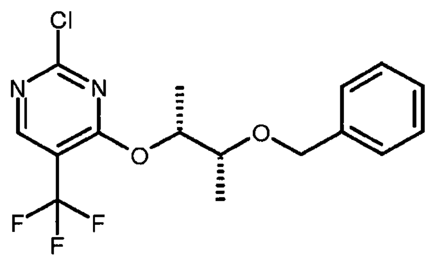

Eine Lösung von 4,66 g (11,1 mmol) 4-((1R,2R)-2-Benzyloxy-1-methyl-propoxy)-2-chlor-5-jod-pyrimidin in 15,8 ml NMP und 15,8 ml THF wurde bei Raumtemperatur unter Rühren mit 3,82 g (20,0 mmol) Kupfer(I)jodid, 0,97 g (16,7 mmol) Kaliumfluorid und 2,47 ml (16,7 mmol) (Trifluormethyl)-trimethylsilan versetzt. Der Ansatz wurde 5,5 Stunden bei 80°C gerührt. Nach dem Erkalten wurde der Ansatz in verdünnte Natriumchlorid-Lösung gegeben und mit Essigester extrahiert (2x). Die vereinten organischen Phasen wurden getrocknet ($Na_2SO_4$), filtriert und eingeengt. Der erhaltene Rückstand wurde chromatographisch (Hexan / Essigester 4:1) gereinigt. Man erhielt 2,17 g (6,0 mmol; Ausbeute: 54%) des Produktes.
$^1$H-NMR (400 MHz, DMSO): $\delta$ = 8.81 (s, 1H), 7.21 (m, 5H), 5.40 (m, 1H), 4.57 (d, 1H), 4.42 (d, 1H), 3.70 (m, 1H), 1.28 (d, 3H), 1.13 (d, 3H).

Verbindung 1.6 wurde alternativ auch durch folgende Vorschrift hergestellt:

Eine Lösung von 5,00 g (23,0 mmol) 2,4-Dichlor-5-trifluormethyl-pyrimidin und 5,40 g (30,0 mmol) (2R,3R)-3-Benzyloxy-butan-2-ol in 60 ml Diethylether und 65 ml Acetonitril wurde bei 0°C mit 1,21 g (27,7 mmol) Natriumhydrd (55%ig), aufgeteilt in 3 Portionen, versetzt und anschließend 90 Minuten bei 15°C gerührt. Der Ansatz wurde mit verdünnter Natriumchlorid-Lösung versetzt und mit Essigester extrahiert (3x). Die vereinten organischen Phasen wurden getrocknet ($Na_2SO_4$), filtriert und eingeengt. Der erhaltene Rückstand wurde chromatographisch (Hexan / Essigester 95:5) gereinigt. Man erhielt 1,60 g (4,4 mmol; Ausbeute: 19%) des Produktes.

**Verbindung 1.7**

**[4-((1R,2R)-2-Benzyloxy-1-methyl-propoxy)-5-trifluormethyl-pyrimidin-2-yl]-(4-cyclopropansulfonyl-phenyl)-amin**

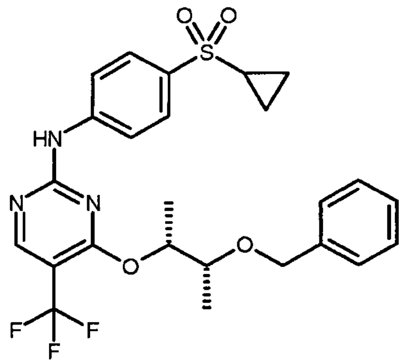

700 mg (1,94 mmol) 4-((1R,2R)-2-Benzyloxy-1-methyl-propoxy)-2-chlor-5-trifluormethyl-pyrimidin und 460 mg (2,33 mmol) 4-Cyclopropansulfonyl-phenylamin in 9,5 ml Acetonitril wurden mit 0,49 ml einer 4N Lösung von Chlorwasserstoff in Dioxan versetzt und 5,5 Stunden bei 80°C gerührt. Nach dem Abkühlen wurde der Ansatz mit Essigester verdünnt und mit gesättigter Natriumhydrogencarbonat-Lösung und gesättigter Natriumchlorid-Lösung gewaschen, getrocknet ($Na_2SO_4$), filtriert und eingeengt. Der erhaltene Rückstand wurde chromatographisch (DCM / Ethanol 95:5) gereinigt. Man erhielt 649 mg (1,24 mmol, Ausbeute: 64%) des Produktes.
$^1$H-NMR (400 MHz, DMSO): $\delta$ = 10.55 (s, 1H), 8.58 (s, 1H), 7.96 (m, 2H), 7.79 (m, 2H), 7.22 (m, 5H), 5.48 (m, 1H), 4.57 (d, 1H), 4.46 (d, 1H), 3.73 (m, 1H), 2.71 (m, 1H), 1.31 (d, 3H), 1.15 (d, 3H), 1.05 (m, 2H), 0.96 (m, 2H).
MS: 522 (ESI+)

**b) Herstellung des Endproduktes**

Eine Lösung von 541 mg (1,04 mmol) [4-((1R,2R)-2-Benzyloxy-1-methyl-propoxy)-5-trifluormethyl-pyrimidin-2-yl]-(4-cyclopropansulfonyl-phenyl)-amin in 110 ml Ethanol wurde mit 541 mg Palladium auf Kohle (10%ig) versetzt und unter Normaldruck bei Raumtemperatur für eine Stunde hydriert. Der Ansatz wurde filtriert und eingeengt. Der erhaltene Rückstand wurde chromatographisch (DCM / EtOH 98:2) gereinigt. Man erhielt 175 mg (0,41 mmol; Ausbeute: 39 %) des Produktes.
$^1$H-NMR (400 MHz, DMSO): $\delta$ = 10.54 (s, 1H), 8.56 (s, 1H), 7.95 (m, 2H), 7.79 (m, 2H), 5.28 (m, 1H), 4.86 (d, 1H), 3.83 (m, 1H), 2.76 (m, 1H), 1.26 (d, 3H), 1.01 (m, 7H).
MS: 432 (ESI+).

**Beispiel 2**

**(2R,3R)-3-[2-(4-Methansulfonyl-phenylamino)-5-trifluormethyl-pyrimidin-4-yloxy]-butan-2-ol**

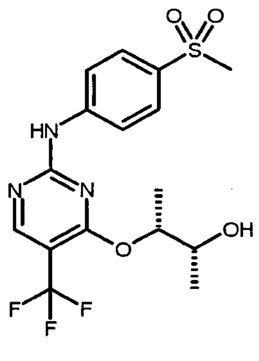

**2a) Herstellung der Zwischenprodukte**

**Verbindung 2.1**

**[4-((1R,2R)-2-Benzyloxy-1-methyl-propoxy)-5-trifluormethyl-pyrimidin-2-yl]-(4-methansulfonyl-phenyl)-amin**

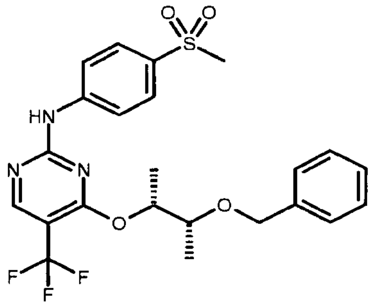

810 mg (2,25 mmol) 4-((1R,2R)-2-Benzyloxy-1-methyl-propoxy)-2-chlor-5-trifluormethyl-pyrimidin und 559 mg (2,69 mmol) 4-Methansulfonyl-phenylamin Hydrochlorid (Acros) in 11 ml Acetonitril wurden 16 Stunden bei 80°C gerührt. Nach dem Abkühlen wurde der Ansatz mit Essigester verdünnt und mit gesättigter Natriumhydrogencarbonat-Lösung und gesättigter NaCl-Lösung gewaschen, getrocknet ($Na_2SO_4$), filtriert und eingeengt. Der erhaltene Rückstand wurde chromatographisch (Hexan / Essigester 1:1) gereinigt. Man erhielt 770 mg (1,55 mmol; Ausbeute: 69%) des Produktes.
$^1$H-NMR (400 MHz, DMSO): $\delta$ = 10.55 (s, 1H), 8.58 (s, 1H), 7.95 (m, 2H), 7.83 (m, 2H), 7.22 (m, 5H), 5.48 (m, 1H), 4.57 (d, 1H), 4.46 (d, 1H), 3.72 (m, 1H), 3.12 (s, 3H), 1.31 (d, 3H), 1.15 (d, 3H). MS: 496 (ESI+)

**b) Herstellung des Endproduktes**

Eine Lösung von 750 mg (1,51 mmol) [4-((1R,2R)-2-Benzyloxy-1-methyl-propoxy)-5-trifluormethyl-pyrimidin-2-yl]-(4-methansulfonyl-phenyl)-amin in 20 ml Ethanol wurde mit 152 mg Palladium auf Kohle (10%ig) versetzt und unter Normaldruck bei Raumtemperatur für 30 Minuten hydriert. Man versetzte erneut mit 152 mg Palladium auf Kohle (10%ig) und hydrierte für 1,5 Stunden. Weitere 152 mg Palladium auf Kohle (10%ig) wurden zugegeben und der Ansatz für 1 Stunde hydriert. Abschließend wurden nochmals 152 mg Palladium auf Kohle (10%ig) zugegeben und 15 Minuten hydriert. Der Ansatz wurde filtriert und eingeengt. Der erhaltene Rückstand wurde chromatographisch (DCM / EtOH 95:5) gereinigt. Man erhielt 512 mg (1,26 mmol; Ausbeute: 83 %) des Produktes.
$^1$H-NMR (400 MHz, DMSO): $\delta$=10.55 (s, 1H), 8.57 (s, 1H), 7.96 (m, 2H), 7.83 (m, 2H), 5.27 (m, 1H), 4.86 (d, 1H), 3.82 (m, 1H), 3.14 (s, 3H), 1.25 (d, 3H), 1.07 (d, 3H).
MS: 405 (EI+).

**Beispiel 3**

**(R)-3-[2-(4-Methansulfonyl-phenylamino)-5-trifluormethyl-pyrimidin-4-yloxy]-2-methyl-butan-2-ol**

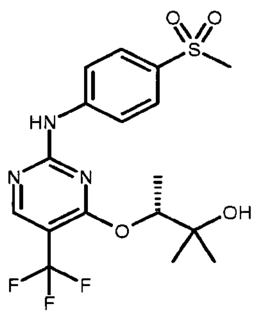

**3a) Herstellung der Zwischenprodukte**

**Verbindung 3.1.**

**(R)-2-Methyl-butan-2,3-diol**

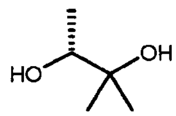

Eine Lösung von 10,0 g (96,1 mmol) (R)-(+)-Milchsäuremethylester in 20 ml THF wurde langsam zu 160 ml (480,0 mmol) einer eisgekühlten 3N Lösung von Methylmagnesiumchlorid in THF getropft. Der Ansatz wurde zunächst langsam auf Raumtemperatur erwärmt und anschließend 30 Minuten refluxiert. Nach dem Abkühlen wurde der Ansatz auf eine gesättigte AmmoniumchloridLösung gegeben und mit Essigester extrahiert (3x). Die vereinten organischen Phasen wurden über einem Whatman Filter filtriert und eingeengt. Man erhielt 4,5 g (43,1 mmol) des Rohproduktes, das ohne weitere Reinigung eingesetzt wurde.
$^{1}$H-NMR (400 MHz, DMSO): δ = 4.21 (d, 1H), 3.93 (s, 1H), 3.29 (m, 1H), 0.97 (m, 9H).

**Verbindung 3.2.**

**(R)-3-(2-Chlor-5-jod-pyrimidin-4-yloxy)-2-methyl-butan-2-ol**

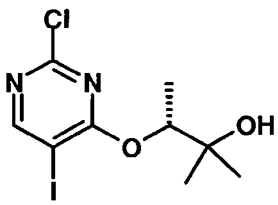

Eine Lösung von 4,40 g (42,3 mmol) (R)-2-Methyl-butan-2,3-diol in 83 ml Diethylether wurde unter Rühren bei 0°C portionsweise mit 1,84 g (42,3 mmol) Natriumhydrid (55%) versetzt und 10 Minuten gerührt. Man rührte weitere 3 Minuten bei Raumtemperatur und gab den Ansatz anschließend zu einer eisgekühlten Lösung von 9,68 g (35,2 mmol) 2,4-Dichlor-5-jod-pyrimidin in 97 ml Acetonitril. Der Ansatz wurde 4 Stunden bei 40°C gerührt und nach dem Erkalten mit Eis und gesättigter NaCl-Lösung versetzt. Man extrahierte mit Essigester (3x). Die vereinten organischen Phasen wurden getrocknet ($Na_2SO_4$), filtriert und eingeengt. Der erhaltene Rückstand wurde chromatographisch (Hexan / Essigester 4:1) gereinigt. Man erhielt 4,96 g (14,5 mmol; Ausbeute: 41%) des Produktes.
$^{1}$H-NMR (400 MHz, DMSO): δ = 8.73 (s, 1H), 4.96 (q, 1H), 4.62 (s, 1H), 1.21 (d, 3H), 1.13 (s, 6H).
ES: 343 (Cl+).

**Verbindung 3.3.**

**2-Chlor-4-[(R)-1,2-dimethyl-2-(tetrahydro-pyran-2-yloxy)-propoxy]-5-jod-pyrimidin**

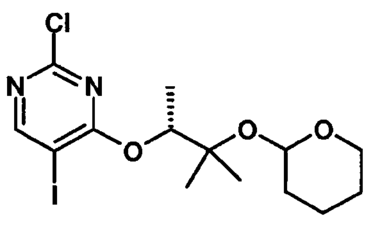

Eine Lösung von 4,96 g (14,5 mmol) (R)-3-(2-Chlor-5-jod-pyrimidin-4-yloxy)-2-methyl-butan-2-ol in 30 ml DCM wurde mit 2,64 ml (29,0 mmol) Dihydropyran und 0,36 g (1,5 mmol) Pyridiniumtosylat versetzt und 22 Stunden bei Raumtemperatur gerührt. Der Ansatz wurde mit DCM verdünnt und mit gesättigter Natriumhydrogencarbonat-Lösung gewaschen. Die organische Phase wurde getrocknet ($Na_2SO_4$), filtriert und eingeengt. Der erhaltene Rückstand wurde chromatographisch (Hexan / Essigester 4:1) gereinigt. Man erhielt 5,50 g (12,9 mmol; Ausbeute: 89%) des Diastereomerengemisches.
$^1$H-NMR (400 MHz, DMSO): $\delta$ = 8.75 (s, 1H), 8.74 (s, 1H), 5.15 (m, 2H), 4.91 (m, 2H), 3.70 (m, 2H), 3.30 (m, 2H), 1.31 (m, 30H).

**Verbindung 3.4.**

**2-Chlor-4-[(R)-1,2-dimethyl-2-(tetrahydro-pyran-2-yloxy)-propoxy]-5-trifluormethyl-pyrimidin**

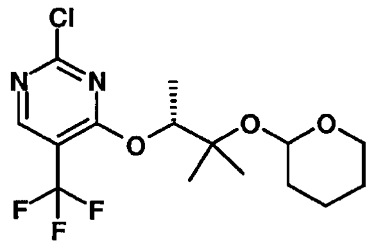

Eine Lösung von 1,00 g (2,34 mmol) 2-Chlor-4-[(R)-1,2-dimethyl-2-(tetrahydro-pyran-2-yloxy)-propoxy]-5-jod-pyrimidin in 3,3 ml NMP und 3,3 ml THF wurde bei Raumtemperatur mit 1,61 g (8,44 mmol) Kupfer(I)jodid, 0,41 g (7,03 mmol) Kaliumfluorid und 1,04 ml (7,03 mmol) (Trifluormethyl)-trimethylsilan versetzt. Der Ansatz wurde 2 Stunden bei 90°C gerührt. Nach dem Erkalten wurde der Ansatz in verdünnte Natriumchlorid-Lösung gegeben und mit Essigester extrahiert (3x). Die vereinten organischen Phasen wurden getrocknet ($Na_2SO_4$), filtriert und eingeengt. Der erhaltene Rückstand wurde chromatographisch (Hexan / Essigester 4:1) gereinigt. Man erhielt 0,53 g (1,43 mmol; Ausbeute: 61%) des Produktes.
$^1$H-NMR (400 MHz, DMSO): $\delta$ = 8.84 (s, 1H), 5.32 (m, 1H), 4.85 (m, 1H), 3.68 (m, 1H), 3.30 (m, 1H), 1.31 (m, 15H).

**b) Herstellung des Endproduktes**

100 mg (0,27 mmol) 2-Chlor-4-[(R)-1,2-dimethyl-2-(tetrahydro-pyran-2-yloxy)-propoxy]-5-trifluormethyl-pyrimidin und 37 mg (0,22 mmol) 4-Methansulfonyl-phenylamin in 2,5 ml Ethanol wurden 150 Minuten bei 70°C gerührt. Der Ansatz wurde zur Trockene eingeengt, mit 3,1 ml Ethanol aufgenommen und mit 12 mg (0,05 mmol) Pyridiniumtosylat versetzt. Der Ansatz wurde 4 Stunden bei 45°C gerührt. Nach dem Erkalten wurde der Ansatz mit verdünnter Natriumhydrogencarbonat-Lösung versetzt und mit Essigester extrahiert (3x). Die vereinten organischen Phasen wurden getrocknet ($Na_2SO_4$), filtriert und eingeengt. Der erhaltene Rückstand wurde chromatographisch (DCM / Ethanol 95:5) gereinigt. Man erhielt 42 mg (0,10 mmol; Ausbeute: 45%) des Produktes.
$^1$H-NMR (400 MHz, DMSO): $\delta$ = 10.55 (s, 1H), 8.57 (s, 1H), 7.96 (m, 2H), 7.84 (m, 2H), 5.14 (q, 1H), 4.66 (s, 1H), 3.14 (s, 3H), 1.28 (d, 3H), 1.12 (s, 6H).
MS: 419 (EI+)

**Beispiel 4**

**(R)-3-[2-(4-Cyclopropansulfonyl-phenylamino)-5-trifluormethyl-pyrimidin-4-yloxy]-2-methyl-butan-2-ol**

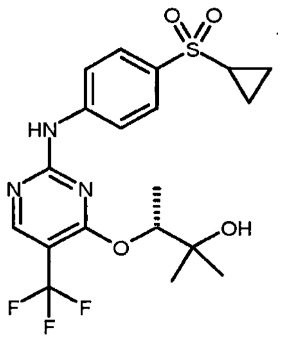

**Herstellung des Endproduktes**

200 mg (0,54 mmol) 2-Chlor-4-[(R)-1,2-dimethyl-2-(tetrahydro-pyran-2-yloxy)-propoxy]-5-trifluormethyl-pyrimidin und 64 mg (0,33 mmol) 4-Cyclopropansulfonyl-phenylamin in 5,0 ml Ethanol wurden 210 Minuten bei 70°C gerührt. Der Ansatz wurde erneut mit 100 mg (0,27 mmol) 2-Chlor-4-[(R)-1,2-dimethyl-2-(tetrahydro-pyran-2-yloxy)-propoxy]-5-trifluormethyl-pyrimidin versetzt und weitere 210 Minuten bei 70°C gerührt. Der Ansatz wurde zur Trockene eingeengt und der erhaltene Rückstand wurde mittels HPLC gereinigt. Man erhielt 91 mg (0,20 mmol; Ausbeute: 61%) des Produktes.

| | | | |
|---|---|---|---|
| Säule: | XBridge C18 5µ 100x30 mm | | |
| Eluent A: | $H_2O$/0.2% $NH_3$ | | |
| Eluent B: | Acetonitril | | |
| Gradient: | 0 min | 50%A | 50%B |
| | 1.00 min | 50%A | 50%B |
| | 7.50 min | 20%A | 80%B |
| | 7.52 min | 1%A | 99%B |
| | 10.00 min | 1%A | 99%B |
| Fluß: | 50,0 mL/min | | |
| Detektor: | DAD scan range 210-400 nm | | |
| | MS ESI+, ESI-, scan range 160-1000 m/z | | |
| Temperatur: | Raumtemperatur | | |
| Retentionszeit: | 4,6-5,5 min | | |

$^1$H-NMR (400 MHz, DMSO): 10.56 (br, 1H), 8.57 (s, 1H), 7.96 (m, 2H), 7.80 (m, 2H), 5.14 (q, 1H), 4.66 (s, 1H), 2.77 (m, 1H), 1.28 (d, 3H), 1.12 (m, 6H), 1.07 (m, 2H), 0.97 (m, 2H).
MS: 445 (EI+)

**Beispiel 5**

**(2R,3R)-3-[2-(4-Benzolsulfonyl-phenylamino)-5-trifluormethyl-pyrimidin-4-yloxy]-butan-2-ol**

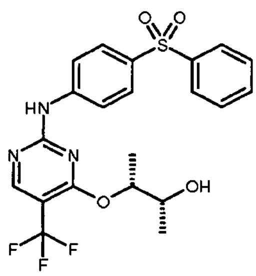

**5a) Herstellung der Zwischenprodukte**

**Verbindung 5.1**

**(4-Benzolsulfonyl-phenyl)-[4-((1R,2R)-2-benzyloxy-1-methyl-propoxy)-5-trifluormethyl-pyrimidin-2-yl]-amin**

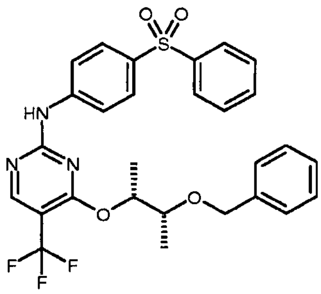

110 mg (0,30 mmol) 4-((1R,2R)-2-Benzyloxy-1-methyl-propoxy)-2-chlor-5-trifluormethyl-pyrimidin und 85 mg (0,37 mmol) 4-Benzolsulfonyl-phenylamin in 1,5 ml Acetonitril wurden mit 0,08 ml einer 4N Lösung von Chlorwasserstoff in Dioxan versetzt und 5 Stunden bei 80°C gerührt. Der Ansatz wurde einrotiert und der erhaltene Rückstand mittels HPLC gereinigt. Man erhielt 121 mg (0,22 mmol, Ausbeute: 71%) des Produktes.

| | | | |
|---|---|---|---|
| System: | Waters Autopurification | | |
| Säule: | XBridge C18 5μ 100x30 mm | | |
| Eluent A: | $H_2O$ / 0.1% HCOOH | | |
| Eluent B: | Acetonitril | | |
| Gradient: | 0 min | 99%A | 1%B |
| | 1.00 min | 99%A | 1%B |
| | 7.50 min | 1%A | 99%B |
| | 10.00 min | 1%A | 99%B |
| Fluß: | 50,0 mL/min | | |
| Detektor: | DAD scan range 210-400 nm | | |
| | MS ESI+, ESI-, scan range 160-1000 m/z | | |
| Temperatur: | Raumtemperatur | | |

$^1$H-NMR (400 MHz, DMSO): δ = 10.56 (s, 1H), 8.56 (s, 1H), 7.89 (m, 6H), 7.58 (m, 3H), 7.22 (m, 5H), 5.44 (m, 1H), 4.55 (d, 1H), 4.44 (d, 1H), 3.71 (m, 1H), 1.29 (d, 3H), 1.14 (d, 3H).

**b) Herstellung des Endproduktes**

Eine Lösung von 116 mg (0,21 mmol) (4-Benzolsulfonyl-phenyl)-[4-((1R,2R)-2-benzyloxy-1-methyl-propoxy)-5-trifluormethyl-pyrimidin-2-yl]-amin in 5 ml Ethanol wurde 2,5 Stunden bei Raumtemperatur unter einer Wasserstoffatmosphäre hydriert. Dabei wurde der Ansatz 6x mit jeweils 50 mg Portionen von Palladium auf Kohle (10%ig) versetzt. Der Ansatz wurde filtriert und eingeengt. Man erhielt 63 mg (0,13 mmol; Ausbeute: 65 %) des Produktes.
$^1$H-NMR (400 MHz, DMSO): δ = 10.55 (s, 1H), 8.55 (s, 1H), 7.89 (m, 6H), 7.59 (m, 3H), 5.25 (m, 1H), 4.86 (d, 1H), 3.80 (m, 1H), 1.24 (d, 3H), 1.06 (d, 3H).
MS: 468 (ESI+)

Die folgenden Beispiele 5-16 fallen nicht unter die Formel des Anspruchs 1 und sind daher nicht erfindungsgemäß.

**Beispiel 6**

**(2R,3R)-3-{2-[4-(Difluor-methansulfonyl)-phenylamino]-5-trifluormethyl-pyrimidin-4-yloxy}-butan-2-ol**

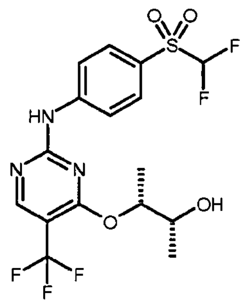

**6a) Herstellung der Zwischenprodukte**

**Verbindung 6.1**

**[4-((1R,2R)-2-Benzyloxy-1-methyl-propoxy)-5-trifluormethyl-pyrimidin-2-yl]-[4-(difluor-methansulfonyl)-phenyl]-amin**

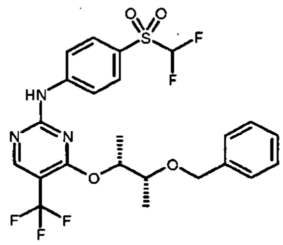

100 mg (0,28 mmol) 4-((1R,2R)-2-Benzyloxy-1-methyl-propoxy)-2-chlor-5-trifluormethyl-pyrimidin und 69 mg (0,33 mmol) 4-(Difluor-methansulfonyl)-phenylamin in 1,4 ml Acetonitril wurden mit 0,07 ml einer 4N Lösung von Chlorwasserstoff in Dioxan versetzt und 3 Stunden bei 80°C gerührt. Der Ansatz wurde einrotiert und der erhaltene Rückstand mittels HPLC gereinigt. Man erhielt 104 mg (0,20 mmol; Ausbeute: 71%) des Produktes.

| | | | |
|---|---|---|---|
| System: | Waters Autopurification | | |
| Säule: | XBridge C18 5μ 100x30 mm | | |
| Eluent A: | $H_2O$ / 0.1% HCOOH | | |
| Eluent B: | Acetonitril | | |
| Gradient: | 0 min | 99%A | 1%B |
| | 1.00 min | 99%A | 1%B |
| | 7.50 min | 1%A | 99%B |
| | 10.00 min | 1%A | 99%B |
| Fluß: | 50,0 mL/min | | |
| Detektor: | DAD scan range 210-400 nm | | |
| | MS ESI+, ESI-, scan range 160-1000 m/z | | |
| Temperatur: | Raumtemperatur | | |

$^1$H-NMR (400 MHz, DMSO): δ = 10.77 (s, 1H), 8.62 (s, 1H), 8.07 (m, 2H), 7.88 (m, 2H), 7.22 (m, 6H), 5.47 (m, 1H), 4.57 (d, 1H), 4.45 (d, 1H), 3.72 (m, 1H), 1.31 (d, 3H), 1.15 (d, 3H).

**b) Herstellung des Endproduktes**

Eine Lösung von 100 mg (0,19 mmol) [4-((1R,2R)-2-Benzyloxy-1-methyl-propoxy)-5-trifluormethyl-pyrimidin-2-yl]-[4-(difluor-methansulfonyl)-phenyl]-amin in 5 ml Ethanol wurde 1,5 Stunden bei Raumtemperatur unter einer Wasserstoffatmosphäre hydriert. Dabei wurde der Ansatz 3x mit jeweils 100 mg Portionen von Palladium auf Kohle (10%ig) versetzt. Der Ansatz wurde filtriert und eingeengt. Man erhielt 45 mg (0,10 mmol; Ausbeute: 54 %) des Produktes.
$^1$H-NMR (400 MHz, DMSO): $\delta$ =10.77 (s, 1H), 8.61 (s, 1H), 8.08 (m, 2H), 7.88 (m, 2H), 7.20 (tr, 1H), 5.29 (m, 1H), 4.88 (d, 1H), 3.84 (m, 1H), 1.26 (d, 3H), 1.07 (d, 3H).
MS: 442 (ESI+)

**Beispiel 7**

**(2R,3R)-3-[2-(4-Cyclopentansulfonyl-phenylamino)-5-trifluormethyl-pyrimidin-4-yloxy]-butan-2-ol**

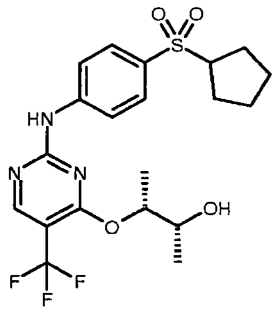

**7a) Herstellung der Zwischenprodukte**

**Verbindung 7.1**

**[4-((1R,2R)-2-Benzyloxy-1-methyl-propoxy)-5-trifluormethyl-pyrimidin-2-yl]-(4-cyclopentansulfonyl-phenyl)-amin**

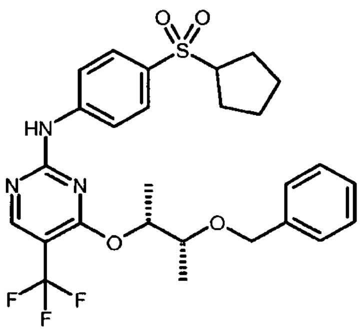

100 mg (0,28 mmol) 4-((1R,2R)-2-Benzyloxy-1-methyl-propoxy)-2-chlor-5-trifluormethyl-pyrimidin und 75 mg (0,33 mmol) 4-Cyclopentansulfonyl-phenylamin in 1,4 ml Acetonitril wurden mit 0,07 ml einer 4N Lösung von Chlorwasserstoff in Dioxan versetzt und 5 Stunden bei 80°C gerührt. Der Ansatz wurde einrotiert und der erhaltene Rückstand mittels HPLC gereinigt. Man erhielt 118 mg (0,21 mmol, Ausbeute: 77%) des Produktes.

| | |
|---|---|
| System: | Waters Autopurification |
| Säule: | XBridge C18 5$\mu$ 100x30 mm |
| Eluent A: | $H_2O$ / 0.1% HCOOH |
| Eluent B: | Acetonitril |

(fortgesetzt)

| | | | |
|---|---|---|---|
| Gradient: | 0 min | 99%A | 1%B |
| | 1.00 min | 99%A | 1%B |
| | 7.50 min | 1%A | 99%B |
| | 10.00 min | 1%A | 99%B |
| Fluß: | 50,0 mL/min | | |
| Detektor: | DAD scan range 210-400 nm | | |
| | MS ESI+, ESI-, scan range 160-1000 m/z | | |
| Temperatur: | Raumtemperatur | | |

$^1$H-NMR (400 MHz, DMSO): δ = 10.57 (s, 1H), 8.58 (s, 1H), 7.96 (m, 2H), 7.77 (m, 2H), 7.22 (m, 5H), 5.48 (m, 1H), 4.56 (d, 1H), 4.46 (d, 1H), 3.72 (m, 1H), 3.63 (m, 1H), 1.76 (m, 4H), 1.51 (m, 4H), 1.30 (d, 3H), 1.14 (d, 3H).

**b) Herstellung des Endproduktes**

Eine Lösung von 112 mg (0,20 mmol) [4-((1R,2R)-2-Benzyloxy-1-methyl-propoxy)-5-trifluormethyl-pyrimidin-2-yl]-(4-cyclopentansulfonyl-phenyl)-amin in 5 ml Ethanol wurde 2,5 Stunden bei Raumtemperatur unter einer Wasserstoffatmosphäre hydriert. Dabei wurde der Ansatz 5x mit jeweils 100 mg Portionen von Palladium auf Kohle (10%ig) versetzt. Der Ansatz wurde filtriert und eingeengt. Man erhielt 75 mg (0,16 mmol; Ausbeute: 80 %) des Produktes.
$^1$H-NMR (400 MHz, DMSO): δ=10.58 (s, 1H), 8.57 (s, 1H), 7.97 (m, 2H), 7.78 (m, 2H), 5.28 (m, 1H), 4.88 (d, 1H), 3.83 (m, 1H), 3.68 (m, 1H), 1.76 (m, 4H), 1.55 (m, 4H), 1.25 (d, 3H), 1.07 (d, 3H).
MS: 460 (ESI+)

**Beispiel 8**

**(R)-2-Methyl-3-{2-[4-(prop-2-en-1-sulfonyl)-phenylamino]-5-trifluormethyl-pyrimidin-4-yloxy}-butan-2-ol**

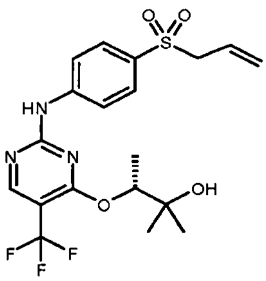

**Herstellung des Endproduktes**

104 mg (0,28 mmol) 2-Chlor-4-[(R)-1,2-dimethyl-2-(tetrahydro-pyran-2-yloxy)-propoxy]-5-trifluormethyl-pyrimidin und 33 mg (0,17 mmol) 4-(Prop-2-en-1-sulfonyl)-phenylamin in 2,6 ml Ethanol wurden 10 Stunden bei 70°C gerührt. Der Ansatz wurde zur Trockene eingeengt und der Rückstand mittels HPLC gereinigt. Man erhielt 12 mg (0,03 mmol; Ausbeute: 10%) des Produktes.

| | | | |
|---|---|---|---|
| System: | Waters Autopurification | | |
| Säule: | XBridge C18 5µ 100x30 mm | | |
| Eluent A: | $H_2O$ / 0.1% HCOOH | | |
| Eluent B: | Acetonitril | | |
| Gradient: | 0 min | 99%A | 1%B |
| | 1.00 min | 99%A | 1%B |
| | 7.50 min | 1%A | 99%B |
| | 10.00 min | 1%A | 99%B |
| Fluß: | 50,0 mL/min | | |

(fortgesetzt)

| | |
|---|---|
| Detektor: | DAD scan range 210-400 nm<br>MS ESI+, ESI-, scan range 160-1000 m/z |
| Temperatur: | Raumtemperatur |

$^1$H-NMR (400 MHz, DMSO): $\delta$ = 10.57 (s, 1H), 8.58 (s, 1H), 7.95 (m, 2H), 7.76 (m, 2H), 5.64 (m, 1H), 5.25 (m, 1H), 5.17 (m, 2H), 4.67 (s, 1H), 4.04 (d, 2H), 1.28 (d, 3H), 1.12 (s, 6H).

**Beispiel 9**

**(2R,3R)-3-{2-[4-(Propan-2-sulfonyl)-phenylamino]-5-trifluormethyl-pyrimidin-4-yloxy}-butan-2-ol**

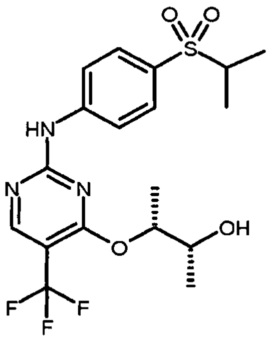

**9a) Herstellung der Zwischenprodukte**

**Verbindung 9.1**

**[4-((1R,2R)-2-Benzyloxy-1-methyl-propoxy)-5-trifluormethyl-pyrimidin-2-yl]-[4-(propan-2-sulfonyl)-phenyl]-amin**

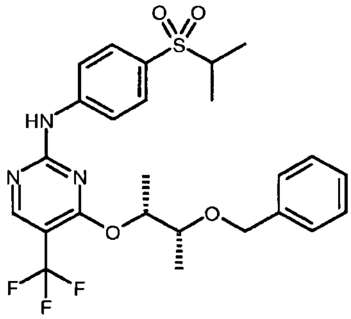

103 mg (0,29 mmol) 4-((1R,2R)-2-Benzyloxy-1-methyl-propoxy)-2-chlor-5-trifluormethyl-pyrimidin und 68 mg (0,34 mmol) 4-(Propan-2-sulfonyl)-phenylamin in 1,4 ml Acetonitril wurden mit 0,07 ml einer 4N Lösung von Chlorwasserstoff in Dioxan versetzt und 5 Stunden bei 80°C gerührt. Der Ansatz wurde einrotiert und der erhaltene Rückstand mittels HPLC gereinigt. Man erhielt 123 mg (0,22 mmol, Ausbeute: 82%) des Produktes.

| | |
|---|---|
| System: | Waters Autopurification |
| Säule: | XBridge C18 5µ 100x30 mm |
| Eluent A: | $H_2O$ / 0.1% HCOOH |
| Eluent B: | Acetonitril |

(fortgesetzt)

| | | | |
|---|---|---|---|
| Gradient: | 0 min | 99%A | 1%B |
| | 1.00 min | 99%A | 1%B |
| | 7.50 min | 1%A | 99%B |
| | 10.00 min | 1%A | 99%B |
| Fluß: | 50,0 mL/min | | |
| Detektor: | DAD scan range 210-400 nm | | |
| | MS ESI+, ESI-, scan range 160-1000 m/z | | |
| Temperatur: | Raumtemperatur | | |

$^1$H-NMR (400 MHz, DMSO): δ = 10.59 (s, 1H), 8.58 (s, 1H), 7.96 (m, 2H), 7.75 (m, 2H), 7.22 (m, 5H), 5.48 (m, 1H), 4.55 (d, 1H), 4.45 (d, 1H), 3.72 (m, 1H), 3.27 (m, 1H), 1.30 (d, 3H), 1.14 (d, 3H), 1.09 (d, 6H).

**b) Herstellung des Endproduktes**

Eine Lösung von 118 mg (0,23 mmol) [4-((1R,2R)-2-Benzyloxy-1-methyl-propoxy)-5-trifluormethyl-pyrimidin-2-yl]-[4-(propan-2-sulfonyl)-phenyl]-amin in 5 ml Ethanol wurde 1,5 Stunden bei Raumtemperatur unter einer Wasserstoffatmosphäre hydriert. Dabei wurde der Ansatz 4x mit jeweils 50 mg Portionen von Palladium auf Kohle (10%ig) versetzt. Der Ansatz wurde filtriert und eingeengt. Man erhielt 43 mg (0,10 mmol; Ausbeute: 44 %) des Produktes.
$^1$H-NMR (400 MHz, DMSO): δ= 10.58 (s, 1H), 8.57 (s, 1H), 7.98 (m, 2H), 7.76 (m, 2H), 5.27 (m, 1H), 4.87 (d, 1H), 3.81 (m, 1H), 3.31 (m, 1H), 1.25 (d, 3H), 1.11 (d, 6H), 1.06 (d, 3H).
MS: 434 (ESI+)

**Beispiel 10**

**(R)-2-Methyl-3-{2-[4-(prop-2-in-1-sulfonyl)-phenylamino]-5-trifluormethyl-pyrimidin-4-yloxy}-butan-2-ol**

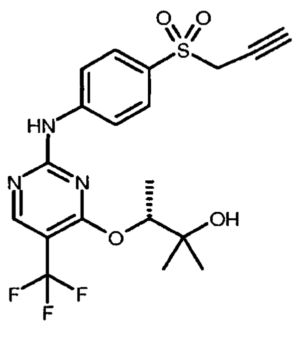

**Herstellung des Endproduktes**

150 mg (0,41 mmol) 2-Chlor-4-[(R)-1,2-dimethyl-2-(tetrahydro-pyran-2-yloxy)-propoxy]-5-trifluormethyl-pyrimidin und 47 mg (0,24 mmol) 4-(Prop-2-in-1-sulfonyl)-phenylamin in 3,6 ml Ethanol wurden 200 Minuten bei 70°C gerührt. Der Ansatz wurde zur Trockene eingeengt. Man erhielt ca. 147 mg des Rohproduktes von dem 60 mg mittels HPLC gereinigt wurden. Man erhielt 31 mg (0,07 mmol) des Produktes.

| | | | |
|---|---|---|---|
| System: | Waters Autopurification | | |
| Säule: | XBridge C18 5µ 100x30 mm | | |
| Eluent A: | $H_2O$ / 0.1% HCOOH | | |
| Eluent B: | Acetonitril | | |
| Gradient: | 0 min | 99%A | 1%B |
| | 1.00 min | 99%A | 1%B |
| | 7.50 min | 1%A | 99%B |

(fortgesetzt)

| | | | |
|---|---|---|---|
| | 10.00 min | 1%A | 99%B |
| Fluß: | 50,0 mL/min | | |
| Detektor: | DAD scan range 210-400 nm | | |
| | MS ESI+, ESI-, scan range 160-1000 m/z | | |
| Temperatur: | Raumtemperatur | | |

$^1$H-NMR (400 MHz, DMSO): δ = 10.60 (s, 1H), 8.58 (s, 1H), 7.98 (m, 2H), 7.83 (m, 2H), 5.14 (q, 1H), 4.67 (s, 1H), 4.45 (d, 2H), 3.38 (tr, 1H), 1.28 (d, 3H), 1.10 (m, 6H).
MS: 444 (ESI+)

**Beispiel 11**

**(2R,3R)-3-{2-[4-(2-Hydroxy-ethansulfonyl)-phenylamino]-5-trifluormethyl-pyrimidin-4-yloxy}-butan-2-ol**

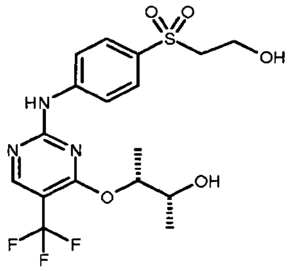

**11a) Herstellung der Zwischenprodukte**

**Verbindung 11.1**

**2-{4-[4-((1R,2R)-2-Benzyloxy-1-methyl-propoxy)-5-trifluormethyl-pyrimidin-2-ylamino]-benzolsulfonyl}-ethanol**

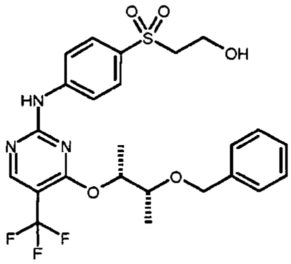

102 mg (0,28 mmol) 4-((1R,2R)-2-Benzyloxy-1-methyl-propoxy)-2-chlor-5-trifluormethyl-pyrimidin und 68 mg (0,34 mmol) 2-(4-Amino-benzolsulfonyl)-ethanol in 1,4 ml Acetonitril wurden mit 0,07 ml einer 4N Lösung von Chlorwasserstoff in Dioxan versetzt und 4 Stunden bei 80°C gerührt. Der Ansatz wurde einrotiert und der erhaltene Rückstand mittels HPLC gereinigt. Man erhielt 72 mg (0,14 mmol, Ausbeute: 48%) des Produktes.

| | | | |
|---|---|---|---|
| System: | Waters Autopurification | | |
| Säule: | XBridge C18 5µ 100x30 mm | | |
| Eluent A: | $H_2O$ / 0.1 % HCOOH | | |
| Eluent B: | Acetonitril | | |
| Gradient: | 0 min | 99%A | 1%B |
| | 1.00 min | 99%A | 1%B |

(fortgesetzt)

| | | | |
|---|---|---|---|
| | 7.50 min | 1%A | 99%B |
| | 10.00 min | 1%A | 99%B |
| Fluß: | 50,0 mL/min | | |
| Detektor: | DAD scan range 210-400 nm | | |
| | MS ESI+, ESI-, scan range 160-1000 m/z | | |
| Temperatur: | Raumtemperatur | | |

$^1$H-NMR (400 MHz, DMSO): δ = 10.56 (s, 1H), 8.58 (s, 1H), 7.94 (m, 2H), 7.79 (m, 2H), 7.22 (m, 5H), 5.46 (m, 1H), 4.83 (tr, 1H), 4.56 (d, 1H), 4.46 (d, 1H), 3.72 (m, 1H), 3.62 (m, 2H), 3.35 (m, 2H), 1.30 (d, 3H), 1.15 (d, 3H).

**b) Herstellung des Endproduktes**

Eine Lösung von 68 mg (0,13 mmol) (2-{4-[4-((1R,2R)-2-Benzyloxy-1-methyl-propoxy)-5-trifluormethyl-pyrimidin-2-ylamino]-benzolsulfonyl}-ethanol in 5 ml Ethanol wurde eine Stunden bei Raumtemperatur unter einer Wasserstoffatmosphäre hydriert. Dabei wurde der Ansatz mit jeweils 68 mg von Palladium auf Kohle (10%ig) versetzt. Der Ansatz wurde filtriert und eingeengt. Man erhielt 28 mg (0,06 mmol; Ausbeute: 50 %) des Produktes.
$^1$H-NMR (400 MHz, DMSO): δ = 10.55 (s, 1H), 8.57 (s, 1H), 7.95 (m, 2H), 7.79 (m, 2H), 5.27 (m, 1H), 4.87 (d, 1H), 4.84 (tr, 1H), 3.82 (m, 1H), 3.63 (m, 2H), 3.36 (m, 2H), 1.25 (d, 3H), 1.07 (d, 3H).
MS: 436 (ESI+)

**Beispiel 12**

**(4-Methansulfonyl-phenyl)-[4-((R)-2-methoxy-1-methyl-ethoxy)-5-trifluormethyl-pyrimidin-2-yl]-amin**

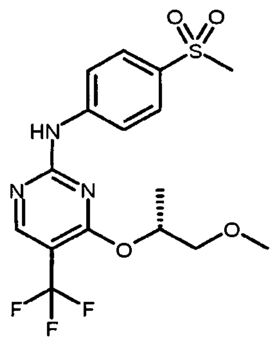

**a) Herstellung der Zwischenprodukte**

**Verbindung 12.1**

**2-Chlor-4-((R)-2-methoxy-1-methyl-ethoxy)-5-trifluormethyl-pyrimidin**

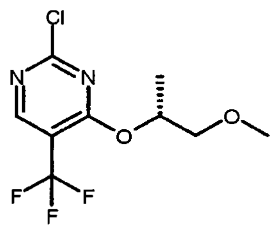

Eine Lösung von 2,00 g (9,2 mmol) 2,4-Dichlor-5-trifluormethyl-pyrimidin und 1,08 g (12,0 mmol) (R)-1-Methoxy-propan-2-ol in 24,4 ml Diethylether und 24,4 ml Acetonitril wurde bei 0°C unter Rühren portionsweise mit 0,48 g Natriumhydrid (55%) versetzt. Der Ansatz wurde im Eisbad langsam auf Raumtemperatur erwärmt. Nach 3,5 Stunden wurde der Ansatz mit Eis und verdünnter Natriumchlorid-Lösung versetzt. Man extrahierte mit Essigester (2x). Die vereinten organischen Phasen wurden getrocknet ($Na_2SO_4$), filtriert und eingeengt. Der erhaltene Rückstand wurde chromatographisch (Hexan / Essigester 7:3) gereinigt. Man erhielt 0,59 g (2,2 mmol; Ausbeute: 24%) des Produktes.
$^1$H-NMR (400 MHz, DMSO): $\delta$ = 8.83 (s, 1H), 5.50 (m, 1H), 3.50 (d, 2H), 3.24 (s, 3H), 1.26 (d, 3H).

**b) Herstellung des Endproduktes**

100 mg (0,37 mmol) 2-Chlor-4-((R)-2-methoxy-1-methyl-ethoxy)-5-trifluormethyl-pyrimidin und 63 mg (0,37 mmol) 4-Methansulfonyl-phenylamin in 2,6 ml Acetonitril wurden mit 0,13 ml einer 4N Lösung von Chlorwasserstoff in Dioxan versetzt und 18 Stunden bei 60°C gerührt. Der Ansatz wurde nach dem Abkühlen mit wenigen Tropfen einer Natriumhydrogencarbonat-Lösung versetzt und einrotiert. Der erhaltene Rückstand wurde mittels HPLC gereinigt. Man erhielt 65 mg (0,16 mmol; Ausbeute: 43%) des Produktes.

| | | | |
|---|---|---|---|
| System: | Waters Autopurification | | |
| Säule: | XBridge C18 5µ 100x30 mm | | |
| Eluent A: | $H_2O$ / 0.1% HCOOH | | |
| Eluent B: | Acetonitril | | |
| Gradient: | 0 min | 99%A | 1%B |
| | 1.00 min | 99%A | 1%B |
| | 7.50 min | 1%A | 99%B |
| | 10.00 min | 1%A | 99%B |
| Fluß: | 50,0 mL/min | | |
| Detektor: | DAD scan range 210-400 nm | | |
| | MS ESI+, ESI-, scan range 160-1000 m/z | | |
| Temperatur: | Raumtemperatur | | |

$^1$H-NMR (400 MHz, DMSO): $\delta$ = 10.57 (s, 1H), 8.58 (s, 1H), 7.93 (m, 2H), 7.82 (m, 2H), 5.51 (m, 1H), 3.52 (m, 2H), 3.26 (s, 3H), 3.14 (s, 3H), 1.30 (d, 3H).

**Beispiel 13**

**(4-Methansulfonyl-phenyl)-(4-prop-2-inyloxy-5-trifluormethyl-pyrimidin-2-yl)-amin**

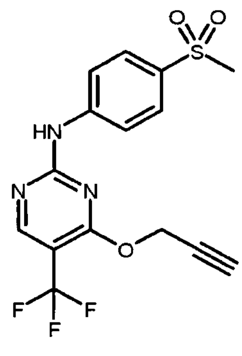

**a) Herstellung der Zwischenprodukte**

**Verbindung 13.1**

**2-Chlor-4-prop-2-inyloxy-5-trifluormethyl-pyrimidin**

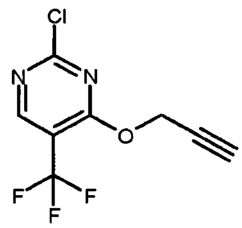

Eine Lösung von 2,00 g (9,2 mmol) 2,4-Dichlor-5-trifluormethyl-pyrimidin und 0,71 ml (12,0 mmol) Prop-2-in-1-ol in 24,4 ml Diethylether und 24,4 ml Acetonitril wurde bei 0°C unter Rühren portionsweise mit 0,48 g Natriumhydrid (55%) versetzt. Der Ansatz wurde im Eisbad langsam auf Raumtemperatur erwärmt. Nach 3,5 Stunden wurde der Ansatz mit Eis und verdünnter Natriumchlorid-Lösung versetzt. Man extrahierte mit Essigester (2x). Die vereinten organischen Phasen wurden getrocknet ($Na_2SO_4$), filtriert und eingeengt. Der erhaltene Rückstand wurde mittels HPLC gereinigt. Man erhielt 0,29 g (1,2 mmol; Ausbeute: 13%) des Produktes.

| | |
|---|---|
| Säule: | Chiralpak IA 5µ 250x30 mm |
| Eluenten: | Hexan / Ethanol 95:5 |
| Fluß: | 40.0 mL/min |
| Detektor: | DAD 210 nm |
| Temperatur: | Raumtemperatur |
| Retentionszeit: | 4,8 - 5,3 min |

$^1$H-NMR (400 MHz, DMSO): δ = 8.91 (s, 1H), 5.18 (d, 2H), 3.71 (tr, 1H).

**b) Herstellung des Endproduktes**

60 mg (0,25 mmol) 2-Chlor-4-prop-2-inyloxy-5-trifluormethyl-pyrimidin und 43 mg (0,25 mmol) 4-Methansulfonyl-phenylamin in 1,8 ml Acetonitril wurden mit 0,06 ml einer 4N Lösung von Chlorwasserstoff in Dioxan versetzt und 18 Stunden bei 60°C gerührt. Der Ansatz wurde einrotiert und der erhaltene Rückstand mittels HPLC gereinigt. Man erhielt 29 mg (0,08 mmol, Ausbeute: 31 %) des Produktes.

| | | | |
|---|---|---|---|
| System: | Waters Autopurification | | |
| Säule: | XBridge C18 5µ 100x30 mm | | |
| Eluent A: | $H_2O$ / 0.1% HCOOH | | |
| Eluent B: | Acetonitril | | |
| Gradient: | 0 min | 99%A | 1%B |
| | 1.00 min | 99%A | 1%B |
| | 7.50 min | 1%A | 99%B |
| | 10.00 min | 1%A | 99%B |
| Fluß: | 50,0 mL/min | | |
| Detektor: | DAD scan range 210-400 nm | | |
| | MS ESI+, ESI-, scan range 160-1000 m/z | | |
| Temperatur: | Raumtemperatur | | |

$^1$H-NMR (400 MHz, DMSO): δ = 10.71 (s, 1H), 8.64 (s, 1H), 7.98 (m, 2H), 7.82 (m, 2H), 5.16 (d, 2H), 3.68 (tr, 1H), 3.14 (s, 3H).

**Beispiel 14**

**(4-Methansulfonyl-phenyl)-[5-trifluormethyl-4-(3,3,3-trifluor-propoxy)-pyrimidin-2-yl]-amin**

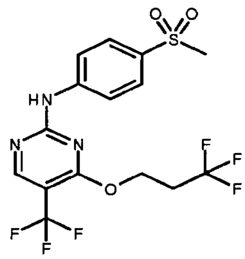

Eine Lösung von 1,00 g (4,63 mmol) 2,4-Dichlor-5-trifluormethyl-pyrimidin und 0,68 g (5,99 mmol) 3,3,3-Trifluor-1-propanol in 12,2 ml Diethylether und 12,2 ml Acetonitril wurde bei 0°C unter Rühren portionsweise mit 0,24 g Natriumhydrid (55%) versetzt. Der Ansatz wurde im Eisbad langsam über Nacht auf Raumtemperatur erwärmt. Der Ansatz wurde mit Eis und verdünnter Natriumchlorid-Lösung versetzt. Man extrahierte mit Essigester (2x). Die vereinten organischen Phasen wurden getrocknet ($Na_2SO_4$), filtriert und eingeengt. Man erhielt 1,50 g des Rohproduktes. 1,03 g des Rohproduktes und 0,59 g (3,48 mmol) 4-Methansulfonyl-phenylamin in 24,1 ml Acetonitril wurden mit 0,87 ml einer 4N Lösung von Chlorwasserstoff in Dioxan versetzt und 24 Stunden bei 60°C gerührt. Der Ansatz wurde mit wenig Natriumhydrogencarbonat-Lösung versetzt und einrotiert. Der erhaltene Rückstand wurde mittels HPLC gereinigt. Man erhielt 0,07 g (0,15 mmol) des Produktes.

| | |
|---|---|
| System: | Waters Autopurification |
| Säule: | XBridge C18 5μ 100x30 mm |
| Eluent A: | $H_2O$ / 0.1% HCOOH |
| Eluent B: | Acetonitril |
| Gradient: | 0-1 min 30% B |
| | 1-7.5 min 30-80% B |
| | 7.5-7.6 min 80-99% B |
| | 7.6-10 min 99% B |
| Fluß: | 50,0 mL/min |
| Detektor: | DAD scan range 210-400 nm |
| | MS ESI+, ESI-, scan range 160-1000 m/z |
| Temperatur: | Raumtemperatur |
| Retentionszeit: | 6,3 - 6,7 min |

$^1$H-NMR (400 MHz, DMSO): δ = 10.66 (s, 1H), 8.65 (s, 1H), 7.99 (m, 2H), 7.88 (m, 2H), 4.72 (tr, 2H), 3.17 (s, 3H), 2.68 (m, 2H).

**Beispiel 15**

**(4-Allyloxy-5-trifluormethyl-pyrimidin-2-yl)-(4-methansulfonyl-phenyl)-amin**

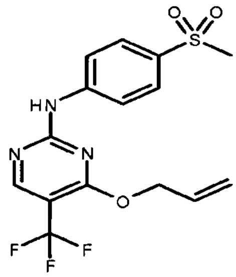

Eine Lösung von 1,00 g (4,63 mmol) 2,4-Dichlor-5-trifluormethyl-pyrimidin und 0,41 ml (5,99 mmol) 2-Propen-1-ol in 12,2 ml Diethylether und 12,2 ml Acetonitril wurde bei 0°C unter Rühren portionsweise mit 0,24 g Natriumhydrid (55%) versetzt. Der Ansatz wurde im Eisbad langsam über Nacht auf Raumtemperatur erwärmt. Der Ansatz wurde mit Eis und verdünnter Natriumchlorid-Lösung versetzt. Man extrahierte mit Essigester (2x). Die vereinten organischen Phasen wurden getrocknet ($Na_2SO_4$), filtriert und eingeengt. Man erhielt 1,14 g des Rohproduktes.

0,76 g des Rohproduktes und 0,55 g (3,19 mmol) 4-Methansulfonyl-phenylamin in 22,0 ml Acetonitril wurden mit 0,80 ml einer 4N Lösung von Chlorwasserstoff in Dioxan versetzt und 24 Stunden bei 60°C gerührt. Der Ansatz wurde mit wenig Natriumhydrogencarbonatlösung versetzt und einrotiert. Der erhaltene Rückstand wurde mittels HPLC gereinigt. Man erhielt 32 mg (0,09 mmol) des Produktes.

| | |
|---|---|
| System: | Waters Autopurification |
| Säule: | XBridge C18 5µ 100x30 mm |
| Eluent A: | $H_2O$ / 0.1% HCOOH |
| Eluent B: | Acetonitril |
| Gradient: | 0-1 min 30% B |
| | 1-7.5 min 30-80% B |
| | 7.5-7.6 min 80-99% B |
| | 7.6-10 min 99% B |
| Fluß: | 50,0 mL/min |
| Detektor: | DAD scan range 210-400 nm |
| | MS ESI+, ESI-, scan range 160-1000 m/z |
| Temperatur: | Raumtemperatur |
| Retentionszeit: | 6,1 - 6,3 min |

$^1$H-NMR (400 MHz, DMSO): $\delta$ = 10.61 (s, 1H), 8.61 (s, 1H), 7.95 (m, 2H), 7.83 (m, 2H), 6.04 (m, 1H), 5.38 (m, 1H), 5.27 (m, 1H), 5.02 (m, 2H), 3.14 (s, 3H).

**Beispiel 16**

**(4-Cyclohexyloxy-5-trifluormethyl-pyrimidin-2-yl)-(4-methansulfonyl-phenyl)-amin**

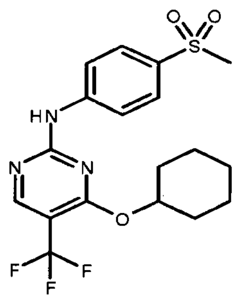

Eine Lösung von 1,00 g (4,63 mmol) 2,4-Dichlor-5-trifluormethyl-pyrimidin und 0,64 ml (5,99 mmol) Cyclohexanol in 12,2 ml Diethylether und 12,2 ml Acetonitril wurde bei 0°C unter Rühren portionsweise mit 0,24 g Natriumhydrid

(55%) versetzt. Der Ansatz wurde im Eisbad langsam über Nacht auf Raumtemperatur erwärmt. Der Ansatz wurde mit Eis und verdünnter Natriumchlorid-Lösung versetzt. Man extrahierte mit Essigester (2x). Die vereinten organischen Phasen wurden getrocknet ($Na_2SO_4$), filtriert und eingeengt. Man erhielt 1,30 g des Rohproduktes.

0,65 g des Rohproduktes und 0,40 g (2,3 mmol) 4-Methansulfonyl-phenylamin in 16,0 ml Acetonitril wurden mit 0,80 ml einer 4N Lösung von Chlorwasserstoff in Dioxan versetzt und 24 Stunden bei 60°C gerührt. Der Ansatz wurde mit wenig Natriumhydrogencarbonatlösung versetzt und einrotiert. Der erhaltene Rückstand wurde mittels HPLC gereinigt. Man erhielt 0,05 g (0,12 mmol) des Produktes.

| | | | |
|---|---|---|---|
| Säule: | XBridge C18 5μ 100x30 mm | | |
| Eluent A: | $H_2O$ | | |
| Eluent B: | Acetonitril | | |
| Gradient: | 0 min | 50%A | 50%B |
| | 1.00 min | 50%A | 50%B |
| | 7.50 min | 20%A | 80%B |
| | 7.52 min | 1%A | 99%B |
| | 10.00 min | 1%A | 99%B |
| Fluß: | 50,0 mL/min | | |
| Detektor: | DAD scan range 210-400 nm | | |
| | MS ESI+, ESI-, scan range 160-1000 m/z | | |
| Temperatur: | Raumtemperatur | | |
| Retentionszeit: | 6,2 - 6,5 min | | |

$^1$H-NMR (400 MHz, DMSO): δ = 10.56 (s, 1H), 8.57 (s, 1H), 7.95 (m, 2H), 7.82 (m, 2H), 5.24 (m, 1H), 3.14 (s, 3H), 1.91 (m, 2H), 1.53 (m, 8H).

**Herstellung der Vergleichsverbindungen**

**Beispiel V1**

**(2R,3R)-3-[2-(4-Cyclopropansulfonyl-phenylamino)-5-trifluormethyl-pyrimidin-4-ylamino]-butan-2-ol**

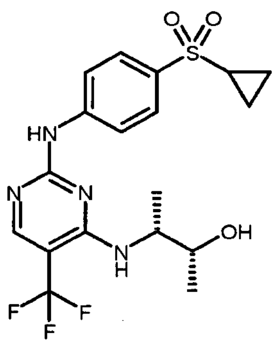

**Herstellung des Endproduktes**

566 mg (2,10 mmol) (2R,3R)-3-(2-Chlor-5-trifluormethyl-pyrimidin-4-ylamino)-butan-2-ol und 414 mg (2,10 mmol) 4-Methansulfonyl-phenylamin in 10,2 ml Acetonitril wurden mit 0,52 ml einer 4N Lösung von Chlorwasserstoff in Dioxan versetzt und 17 Stunden bei 60°C gerührt. Der Ansatz wurde einrotiert und der erhaltene Rückstand chromatographisch (DCM / Ethanol 95:5) gereinigt. Man erhielt 670 mg (1,56 mmol, Ausbeute: 74%) des Produktes.
$^1$H-NMR (400 MHz, DMSO): δ = 10.13 (s, 1H), 8.27 (s, 1H), 7.97 (m, 2H), 7.75 (m, 2H), 6.08 (d, 1H), 5.07 (d, 1H), 4.14 (m, 1H), 3.77 (m, 1H), 2.74 (m, 1H), 1.20 (d, 3H), 1.00 (m, 7H).
MS: 431 (ESI+)

**Beispiel V2**

**(2R,3R)-3-[2-(4-Methansulfonyl-phenylamino)-5-trifluormethyl-pyrimidin-4-ylamino]-butan-2-ol**

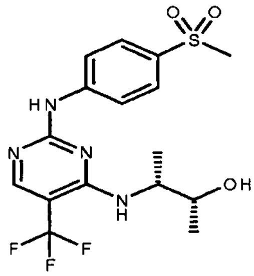

**a) Herstellung der Zwischenprodukte**

**Verbindung V2.1**

**(2R,3R)-3-(2-Chlor-5-tritluormethyl-pyrimidin-4-ylamino)-butan-2-ol Trifluoracetat**

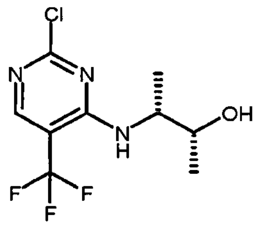

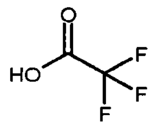

7,60 g (35,0 mmol) 2,4-Dichlor-5-trifluormethyl-pyrimidin und 4,40 g (35,0 mmol) (2R,3R)-3-Amino-butan-2-ol Hydrochlorid in 139 ml Acetonitril wurden bei 0°C unter Rühren tropfenweise mit 9,71 ml (70,0 mmol) Triethylamin versetzt. Der Ansatz wurde im Eisbad langsam auf Raumtemperatur erwärmt. Nach 3 Tagen wurde der Ansatz in halbkonzentrierte Natriumchlorid-Lösung gegeben. Man extrahierte mit Essigester (2x). Die vereinten organischen Phasen wurden getrocknet ($Na_2SO_4$), filtriert und eingeengt. Der erhaltene Rückstand wurde mittels HPLC gereinigt. Man erhielt 2,49 g (6,5 mmol; Ausbeute: 19%) des Produktes.

| | |
|---|---|
| Säule: | XBridge C18 5μ 100x30 mm |
| Eluenten: | A: $H_2O$ B: MeCN |
| Puffer: | A / 0,1% TFA |
| Gradient: | 60% A + 40% B (2')_40→ 70% B (5,5') → 99% B (0,1') |
| Fluß: | 50,0 mL/min |
| Detektor: | DAD (200-400 nm) TAC; MS-ESI+ (125-925 m/z) TIC |
| Temperatur: | Raumtemperatur |
| Retentionszeit: | 3,1 - 3,8 min |

$^1$H-NMR (400 MHz, DMSO): δ = 8.38 (s, 1H), 6.73 (d, 1H), 4.07 (m, 1H), 3.71 (m, 1H), 1.12 (d, 3H), 1.01 (d, 3H).

**b) Herstellung des Endproduktes**

84 mg (0,22 mmol) (2R,3R)-3-(2-Chlor-5-trifluormethyl-pyrimidin-4-ylamino)-butan-2-ol Trifluoracetat und 46 mg (0,22 mmol) 4-Methansulfonyl-phenylamin Hydrochlorid in 1,4 ml Acetonitril wurden mit 0,06 ml einer 4N Lösung von Chlorwasserstoff in Dioxan versetzt und bei 50°C gerührt. Nach 19 Stunden wurden erneut 21 mg (0,06 mmol)

(2R,3R)-3-(2-Chlor-5-trifluormethyl-pyrimidin-4-ylamino)-butan-2-ol Trifluoracetat zugegeben und weitere 24 Stunden bei 50°C gerührt. Der Ansatz wurde einrotiert und der erhaltene Rückstand chromatographisch (DCM / Ethanol 95:5) gereinigt. Man erhielt 42 mg (0,10 mmol, Ausbeute: 45%) des Produktes.
$^1$H-NMR (400 MHz, DMSO): δ = 10.13 (s, 1H), 8.26 (s, 1H), 7.96 (m, 2H), 7.78 (m, 2H), 6.08 (d, 1H), 5.07 (br, 1H), 4.14 (m, 1H), 3.76 (m, 1H), 3.12 (s, 3H), 1.20 (d, 3H), 1.05 (d, 3H).
MS: 405 (ESI+)

**Beispiel V3**

**(R)-3-[2-(4-Methansulfonyl-phenylamino)-5-trifluormethyl-pyrimidin-4-ylamino]-2-methyl-butan-2-ol**

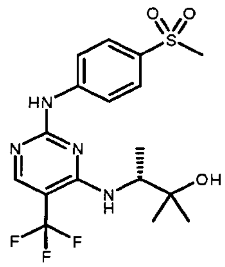

**a) Herstellung der Zwischenprodukte**

**Verbindung V3.1**

**(R)-3-(2-Chlor-5-trifluormethyl-pyrimidin-4-ylamino)-2-methyl-butan-2-ol**

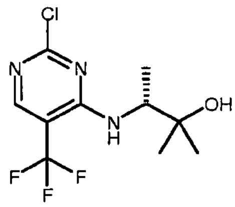

12,4 g (57,4 mmol) 2,4-Dichlor-5-trifluormethyl-pyrimidin und 5,90 g (57,3 mmol) (R)-3-Amino-2-methyl-butan-2-ol in 227 ml Acetonitril wurden bei 0°C unter Rühren tropfenweise mit 15,85 ml (114,4 mmol) Triethylamin versetzt. Der Ansatz wurde im Eisbad langsam auf Raumtemperatur erwärmt. Nach 18 Stunden wurde der Ansatz in halbkonzentrierte Natriumchlorid-Lösung gegeben. Man extrahierte mit Essigester (2x). Die vereinten organischen Phasen wurden getrocknet ($Na_2SO_4$), filtriert und eingeengt. Der erhaltene Rückstand wurde mittels HPLC gereinigt. Man erhielt 6,39 g (22,5 mmol; Ausbeute: 39%) des Produktes.

| | | | |
|---|---|---|---|
| Säule: | XBridge C18 5μ 150x30 mm | | |
| Eluent A: | $H_2O$ / 0.2% $NH_3$ | | |
| Eluent B: | Acetonitril | | |
| Gradient: | 0 min | 99% A | 1% B |
| | 1.00 min | 99% A | 1% B |
| | 7.50 min | 1% A | 99% B |
| | 10.00 min | 1% A | 99% B |
| Fluß: | 50,0 mL/min | | |
| Detektor: | DAD scan range 200-400 nm | | |
| | MS ESI+, ESI-, scan range 120-1000 m/z | | |

(fortgesetzt)

| | |
|---|---|
| Temperatur: | Raumtemperatur |
| Retentionszeit: | 6,9 - 8.3 min |

$^1$H-NMR (400 MHz, DMSO): δ = 8.45 (s, 1H), 6.55 (d, 1H), 5.00 (s, 1H), 4.11 (m, 1H), 1.14 (m, 9H).

**b) Herstellung des Endproduktes**

199 mg (0,70 mmol) (R)-3-(2-Chlor-5-trifluormethyl-pyrimidin-4-ylamino)-2-methyl-butan-2-ol und 146 mg (0,70 mmol) 4-Methansulfonyl-phenylamin Hydrochlorid in 3,4 ml Acetonitril wurden 16 Stunden bei 60°C gerührt. Der Ansatz wurde einrotiert und der erhaltene Rückstand chromatographisch (DCM / Ethanol 95:5) gereinigt. Man erhielt 151 mg (0,36 mmol, Ausbeute: 51%) des Produktes.
$^1$H-NMR (400 MHz, DMSO): δ = 10.14 (s, 1H), 8.27 (s, 1H), 7.96 (m, 2H), 7.78 (m, 2H), 6.06 (d, 1H), 4.91 (br, 1H), 4.11 (m, 1H), 3.12 (s, 3H), 1.11 (m,9H).
MS: 419 (ESI+)

**Beispiel 17**

**Assay 1: CDK1/CycB Kinase Assay**

Rekombinante CDK1- und CycB-GST-Fusionsproteine, gereinigt aus Bakulovirus-infizierten Insektenzellen (Sf9), wurden von ProQinase GmbH, Freiburg, gekauft. Das als Kinase-Substrat verwendet Histon IIIS ist über die Fa. Sigma käuflich zu erwerben.

CDK1/CycB (200 ng/Messpunkt) wurde für 10 min bei 22°C in Anwesenheit verschiedener Konzentrationen an Testsubstanzen (0 μM, sowie innerhalb des Bereiches 0,001 - 10 μM) in Assaypuffer [50 mM Tris/HCl pH8,0; 10 mM $MgCl_2$; 0,1 mM Na ortho-Vanadat; 1,0 mM Dithiothreitol; 0,5 μM Adenosintrisphosphat (ATP); 10 μg/Messpunkt Histon IIIS; 0,2 μCi/Messpunkt $^{33}$P-gamma ATP; 0,05% NP40; 1,25% Dimethylsulfoxid] inkubiert. Die Reaktion wurde durch Zugabe von EDTA-Lösung (250 mM; pH8,0; 15 μl/Messpunkt) gestoppt.

Von jedem Reaktionsansatz wurden 15 μl auf P30 Filterstreifen (Fa. Wallac) aufgetragen, und nicht-eingebautes $^{33}$P-ATP wurde durch dreimaliges Waschen der Filterstreifen für je 10 min in 0,5%iger Phosphorsäure entfernt. Nach dem Trocknen der Filterstreifen für 1 Stunde bei 70°C wurden die Filterstreifen mit Szintillator-Streifen (MeltiLex™ A, Fa. Wallac) bedeckt und für 1 Stunde bei 90°C eingebrannt. Die Menge an eingebautem $^{33}$P (Substratphosphorylierung) wurde durch Szintillationsmessung in einem Gamma-Strahlungsmessgerät (Wallac) bestimmt. Die Messdaten wurden normalisiert auf 0% Inhibition (Enzymreaktion ohne Inhibitor) und 100% Inhibition (alle Assaykomponenten außer Enzym). Die Bestimmung der IC50 Werte erfolgte mittels eines 4-Parameter Fits unter Benutzung firmeneigener Software.

**Assay 2: CDK2/CycE Kinase Assay**

Rekombinante CDK2- und CycE-GST-Fusionsproteine, gereinigt aus Bakulovirus-infizierten Insektenzellen (Sf9), wurden von ProQinase GmbH, Freiburg, gekauft. Histon IIIS, das als Kinase-Substrat verwendet wurde, wurde bei der Fa. Sigma gekauft.

CDK2/CycE (50 ng/Messpunkt) wurde für 10 min bei 22°C in Anwesenheit verschiedener Konzentrationen an Testsubstanzen (0 μM, sowie innerhalb des Bereiches 0,001 - 10 μM) in Assaypuffer [50 mM Tris/HCl pH8,0; 10 mM $MgCl_2$; 0,1 mM Na ortho-Vanadat; 1,0 mM Dithiothreitol; 0,5 μM Adenosintrisphosphat (ATP); 10 μg/Messpunkt Histon IIIS; 0,2 μCi/Messpunkt $^{33}$P-gamma ATP; 0,05% NP40; 1,25% Dimethylsulfoxid] inkubiert. Die Reaktion wurde durch Zugabe von EDTA-Lösung (250 mM; pH8,0; 15 μl/Messpunkt) gestoppt.

Von jedem Reaktionsansatz wurden 15 μl auf P30 Filterstreifen (Fa. Wallac) aufgetragen, und nicht-eingebautes $^{33}$P-ATP wurde durch dreimaliges Waschen der Filterstreifen für je 10 min in 0,5%iger Phosphorsäure entfernt. Nach dem Trocknen der Filterstreifen für 1 Stunde bei 70°C wurden die Filterstreifen mit Szintillator-Streifen (MeltiLex™ A, Fa. Wallac) bedeckt und für 1 Stunde bei 90°C eingebrannt. Die Menge an eingebautem $^{33}$P (Substratphosphorylierung) wurde durch Szintillationsmessung in einem Gamma-Strahlungsmessgerät (Wallac) bestimmt. Die Messdaten wurden normalisiert auf 0% Inhibition (Enzymreaktion ohne Inhibitor) und 100% Inhibition (alle Assaykomponenten außer Enzym). Die Bestimmung der IC50 Werte erfolgte mittels eines 4-Parameter Fits unter Benutzung firmeneigener Software.

**Assay 3: CDK4/CycD Kinase Assay**

Rekombinante CDK4- und CycD1-GST-Fusionsproteine, gereinigt aus Bakulovirus-infizierten Insektenzellen

(Sf9), wurden von ProQinase GmbH, Freiburg, gekauft. CDK4/CycD1 (250 ng/Messpunkt) wurde für 3 Stunden bei 22°C in Anwesenheit verschiedener Konzentrationen an Testsubstanzen (0 μM, sowie innerhalb des Bereiches 0,001 - 10 μM) in 31 μl Assaypuffer [50 mM Hepes pH7,0; 2,5 mM MnCl; 0,05 mM Na ortho-Vanadat; 1,0 mM Dithiothreitol; 0,25 μM Adenosintrisphosphat (ATP); 0,5 μM biotinyliertes myelin basic protein (bio-MBP, GE Healthcare); 0,05 μCi/Messpunkt $^{33}$P-gamma ATP; 0,005% NP40; 0,025% bovines Serumalbumin; 3% Dimethylsulfoxid] inkubiert. Die Reaktion wurde durch Zugabe von 50 μl Stopp-Mix [100 μM ATP; 10 mM EDTA pH8,0; 0.2% Triton X100; 0.125 mg Streptavidin-SPA Beads (GE Healthcare)] abgestoppt. Nach 10 min Inkubation bei Raumtemperatur wurden die SPA Beads durch Zentrifugation (10 min; 1500g) pelletiert. Die Menge an eingebautem $^{33}$P (Substratphosphorylierung) wurde durch Szintillationsmessung in einem beta-Strahlungsmessgerät (Microbeta, Perkin Elmer) bestimmt. Die Messdaten wurden normalisiert auf 0% Inhibition (Enzymreaktion ohne Inhibitor) und 100% Inhibition (alle Assaykomponenten außer Enzym).

Die Bestimmung der IC50 Werte erfolgte mittels eines 4-Parameter Fits unter Benutzung firmeneigener Software.

**Assay 4: VEGF Rezeptor-2 Kinase Assay**

Rekombinante VEGF Rezeptortyrosinkinase-2 wurde als GST-Fusionsprotein aus Bakulovirus-infizierten Insektenzellen (Sf9) gereinigt. Poly-(Glu4Tyr), das als Kinase-Substrat verwendet wurde, wurde bei der Fa. Sigma gekauft.

VEGF Rezeptortyrosinkinase (90 ng/Messpunkt) wurde für 10 min bei 22°C in Anwesenheit verschiedener Konzentrationen an Testsubstanzen (0 μM, sowie innerhalb des Bereiches 0,001 - 10 μM) in 30 μl Assaypuffer [40 mM Tris/HCl pH5,5; 10 mM $MgCl_2$; 1 mM $MnCl_2$; 3 μM Na ortho-Vanadat; 1,0 mM Dithiothreitol; 8 μM Adenosintrisphosphat (ATP); 0,96 μg/Messpunkt poly-($Glu_4$Tyr); 0,2 μCi/Messpunkt $^{33}$P-gamma ATP; 1.4% Dimethylsulfoxid] inkubiert. Die Reaktion wurde durch Zugabe von EDTA-Lösung (250 mM; pH8,0; 15 μl/Messpunkt) gestoppt.

Von jedem Reaktionsansatz wurden 15 μl auf P30 Filterstreifen (Fa. Wallac) aufgetragen, und nicht-eingebautes $^{33}$P-ATP wurde durch dreimaliges Waschen der Filterstreifen für je 10 min in 0,5%iger Phosphorsäure entfernt. Nach dem Trocknen der Filterstreifen für 1 Stunde bei 70°C wurden die Filterstreifen mit Szintillator-Streifen (MeltiLex™ A, Fa. Wallac) bedeckt und für 1 Stunde bei 90°C eingebrannt. Die Menge an eingebautem $^{33}$P (Substratphosphorylierung) wurde durch Szintillationsmessung in einem gamma-Strahlungsmessgerät (Wallac) bestimmt. Die Messdaten wurden normalisiert auf 0% Inhibition (Enzymreaktion ohne Inhibitor) und 100% Inhibition (alle Assaykomponenten außer Enzym). Die Bestimmung der IC50 Werte erfolgte mittels eines 4-Parameter Fits unter Benutzung firmeneigener Software.

**Assay 5: Proliferationsassays**

Kultivierte humane Tumorzellen (MCF7, hormonunabhängige menschliche Mammakarzinomazellen, bezogen von ATCC HTB22; NCI-H460, menschliche nicht kleinzellige Lungenkarzinomazellen, ATCC HTB-177; DU 145, hormonunabhängige menschliche Prostatakarzinomzellen, ATCC HTB-81; HeLa-MaTu, menschliche Cervix-Karzinomzellen, EPO-GmbH, Berlin; HeLa-MaTu-MDR, multiple Arzneimittel resistente menschliche Cervix-Karzinomzellen, EPO-GmbH, Berlin; Caco-2, menschliche Colon-Karzinomazellen, ATCC HTB-37; B16F10, murine Melanomzelle, ATCC CRL-6475) wurden in einer Dichte von ca. 1000-5000 Zellen/Messpunkt, je nach Wachstumsgeschwindigkeit der jeweiligen Zellen, in einer 96-Loch Multititerplatte in 200 μl des entsprechenden Wachstumsmediums ausplattiert. Nach 24 Stunden wurden die Zellen einer Platte (Nullpunkt-Platte) mit Kristallviolett gefärbt (s.u.), während das Medium der anderen Platten durch frisches Kulturmedium (200 μl), dem die Testsubstanzen in verschiedenen Konzentrationen (0 μM, sowie im Bereich 0,003 - 3 μM; die finale Konzentration des Lösungsmittels Dimethylsulfoxid betrug 0,5%) zugesetzt waren, ersetzt. Die Zellen wurden für 4 Tage in Anwesenheit der Testsubstanzen inkubiert. Die Zellproliferation wurde durch Färbung der Zellen mit Kristallviolett bestimmt: Die Zellen wurden durch Zugabe von 20 μl/Messpunkt einer 11%igen Glutaraldehyd-Lösung 15 min bei Raumtemperatur fixiert. Nach dreimaligem Waschen der fixierten Zellen mit Wasser wurden die Platten bei Raumtemperatur getrocknet. Die Zellen wurden durch Zugabe von 100 μl/Messpunkt einer 0,1%igen Kristallviolett-Lösung (pH durch Zugabe von Essigsäure auf pH3 eingestellt) gefärbt. Nach dreimaligem Waschen der gefärbten Zellen mit Wasser wurden die Platten bei Raumtemperatur getrocknet. Der Farbstoff wurde durch Zugabe von 100 μl/Messpunkt einer 10%igen Essigsäure-Lösung gelöst. Die Extinktion wurde photometrisch bei einer Wellenlänge von 595 nm bestimmt. Die Messdaten wurden normalisiert auf 0% Inhibition [unbehandelte (0 μM) Zellen] und 100% Inhibition (Extinktionwerte der Nullpunktplatte). Die Bestimmung der IC50 Werte erfolgte mittels eines 4-Parameter Fits unter Benutzung firmeneigener Software.

**Assay 6: Permeabilitätsassays**

Der Caco-2 Monolayer stellt eine Barriere zwischen 2 Kompartimenten dar. Die Zellen verhalten sich dabei ähnlich der Dünndarmzellen. Wirkstoffe können sowohl para- wie auch transzellulär transportiert werden. Dabei erfolgt der Transport meist von *apikal* (*luminal*) nach *basolateral* (*serosal*). Bei p-Glycoproteinsubstraten wird in der Regel auch

der Rücktransport von *basolateral* nach *apikal* beobachtet.

Versuchsdurchführung: Der Permeabilitätsversuch wird sowohl von *apikal* nach *basolateral* als auch von *basolateral* nach *apikal* durchgeführt. Dabei werden pro Substanz 2 Filter verwendet; die Inkubation erfolgt über 90 min bei 37°C im Wasserbad. Neben der bidirektionalen Permeabilität von Test- und Referenzsubstanzen (Referenzen: geringe Permeabilität: PEG4000; hohe Permeabilität: Clonidin; direktionale Permeabilität: Digoxin), wird durch die Bestimmung des transepithelialen Widerstandes (TEER) die Integrität des Zellmonolayers sichergestellt. Als Referenzsubstanzen werden (i) PEG 4000 als hydrophiler Marker benutzt. Es ist wegen seines hohen Molekulargewichtes unfähig in die Zellmembran oder in die Poren der Tight junctions zu permeieren. PEG 4000 ist daher ein Marker für die Unversehrtheit des Zellmonolayers und die Enge der Tight junctions. PEG 4000 wird im Menschen nicht absorbiert. (ii) Clonidine ist bekannt als vollständig absorbierte Substanzen beim Menschen (100 %). Sie dienen als Marker für sehr gutpermeable Substanzen mit Papp-Werten über 100 nm/s. (iii) Digoxin ist ein bekanntes Pgp Substrat. Es zeigt eine geringe Permeabilität von *apikal* nach *basolateral.* Beim Rückversuch (*basolateral* nach *apikal*) sollten die Papp-Werte bedingt durch den aktiven direktionalen Transport ins *apikale* Kompartment um ca. Faktor 10 höher liegen.

Auswertung: Der Permeationskoeffizient (Papp) wird über die Substanzkonzentration auf der Donor- und Rezeptorseite nach folgender Formel berechnet:

$$\text{Papp} = (\text{Vc res} / \text{A} * \text{C}_{\text{t0, don}}) * (\text{delta C}_{\text{res}}/\text{delta T}),$$

wobei

| | |
|---|---|
| Vres: | Puffervolumen auf der Rezeptorseite, |
| A: | Filterfläche = 1 $cm^2$, |
| $C_{t0,\ don}$: | Substanzkonzentration auf der Donorseite zum Zeitpunkt 0, |
| delta $C_{res}$/delta T: | Änderung der Substanzkonzentration über die Zeit auf der Rezeptorseite. |

Der Permeationskoeffizient Papp wird zur Einschätzung der Absorption beim Menschen nach folgendem Schema genutzt:

| Permeationskoeffizient Papp [nm/s] | geschätzte Absorption |
|---|---|
| >1 / <10 | Schlechte Absorption, bevorzugt parazellulär über die tight junctions (Bsp.: Mannitol, Sucrose, Cimetidine) |
| >10 / <60 | Mittelmäßige Absorption, bevorzugt transzellulär |
| >60 / >100 | Gute Absorption, bevorzugt transzellulär (Bsp.: Clonidine, Testosteron) |

**Ergebnisse aus den Enzym- und Zellassays**

Tab.1: Ergebnisse der Enzymassays

| Bsp. | CDK1/CycB (Assay 1) | CDK2/CycE (Assay 2) | CDK4/CycD (Assay 3) | VEGF-R2 (Assay 4) |
|---|---|---|---|---|
| | Konzentration der halbmaximalen Inhibition der Enzymaktivität, IC50 [nM] | | | |
| 1 | 16 | 16 | 20 | 260 |
| 2 | 9 | 8 | 24 | 480 |
| 3 | 32 | 12 | 78 | 490 |
| 4 | 36 | 21 | | 180 |
| 5 | 41 | 36 | | 350 |
| 6 | 24 | 12 | | 430 |
| 7 | 23 | 21 | | 230 |
| 8 | 43 | 11 | | 460 |

(fortgesetzt)

| Bsp. | CDK1/CycB (Assay 1) | CDK2/CycE (Assay 2) | CDK4/CycD (Assay 3) | VEGF-R2 (Assay 4) |
|---|---|---|---|---|
| 9 | 12 | 6 | | 170 |
| 10 | 82 | 43 | | 200 |
| 11 | 3 | 3 | | 170 |
| 12 | 51 | 12 | | 400 |
| 13 | 130 | 25 | | 610 |
| 14 | 740 | 94 | | >1000 |
| 15 | 280 | 49 | | 850 |
| 16 | >1000 | 480 | | 890 |
| | | | | |
| Struktur 692 des Beispiels 1 aus WO 2003/032997 | 53 | 55 | >1000 | 120 |
| Vergleichs-beispiel V1 | 8 | 6 | 52 | 51 |
| Vergleichs-beispiel V2 | 4 | 4 | 35 | 92 |
| Vergleichs-beispiel V3 | 9 | 8 | 24 | 190 |

Tab.2: Ergebnisse der Proliferationsassays (Assay 5)

| Bsp. | HeLa-MaTu | HeLa-MaTu-ADR | MCF7 | NCI-H460 | DU145 | Caco-2 | B16F10 |
|---|---|---|---|---|---|---|---|
| | Konzentration der halbmaximalen Inhibition der Zellproliferation, IC50 [nM] | | | | | | |
| 1 | 11 | 9 | 23 | 41 | 17 | 45 | 49 |
| 2 | 14 | 14 | 35 | 38 | 34 | 66 | 70 |
| 3 | 35 | 39 | 160 | 140 | 74 | 180 | 270 |
| 4 | | 142 | 105 | 226 | 194 | 205 | 287 |
| 12 | | | 191 | | | | |
| 13 | | | 740 | | | | |
| 14 | | | 2200 | | | | |
| 15 | | | 1500 | | | | |
| 16 | | | 1400 | | | | |
| | | | | | | | |
| Struktur 692 des Beispiels 1 aus WO 2003/032997 | 150 | 1200 | 71 | 290 | 510 | 1400 | 450 |
| Vergleichs-beispiel V1 | | 26 | 65 | 37 | 46 | 63 | 78 |
| Vergleichsbeispiel V2 | | 32 | 43 | 34 | 75 | 76 | 89 |
| Vergleichsbeispiel V3 | | 37 | 61 | 93 | 60 | 133 | 106 |

Tab.3: Ergebnisse der Permeabilitätsassays (Assay 6)

| Bsp. | apikal-basolateral (a-b) | basolateral-apikal (b-a) | ratio (b-a)/(a-b) |
|---|---|---|---|
| | Permeationskoeffizient, Papp [nM/s] | | |
| 1 | 165 | 147 | 0.9 |
| 2 | 196 | 194 | 1.0 |
| | | | |
| Struktur 692 des Beispiels 1 aus WO 2003/032997 | 9 | 294 | 32 |
| Vergleichsbeispiel V1 | 207 | 174 | 0,8 |
| Vergleichsbeispiel V2 | 180 | 178 | 1.0 |

**Schlußfolgerungen aus den Enzym- und Zellassays**

Die Beispielverbindungen 1-3 zeigen eine 2- bis 6-fach stärke Inhibition der Aktivität der Zyklin-abhängigen Kinase CDK1 und eine 3- bis 7-fach stärkere Inhibition der CDK2 im Vergleich zu der Struktur 692 des Beispiels 1 aus WO 2003/032997 (Tab.1). Die Beispielverbindungen 1-3 zeigen eine potente Inhibition der CDK4 bei nanomolaren Konzentrationen während die Struktur 692 des Beispiels 1 aus WO 2003/032997 die halb-maximale Hemmung der CDK4 Aktivität bei zu einer Konzentration von 1000 nM noch nicht erreicht hat. Gleichzeitig ist die Selektivität hinsichtlich der CDK-Inhibition gegenüber der Inhibition der VEGF-Rezeptorkinase-2 (VEGF-R2) bei den Beispielen 1-3 deutlich erhöht (15- bis 50-fach stärkere inhibition der CDKs), während die Struktur 692 des Beispiels 1 aus WO 2003/032997 nur eine ca. 2-fache Selektivität aufweist. In den Zellproliferationsassays zeigen die Beispielverbindungen 1-3 die 50%ige Inhibition der Proliferation bereits bei deutlich niedrigeren Konzentrationen als die Struktur 692 des Beispiels 1 aus WO 2003/032997 (Tab. 2, Ausnahme: Bsp. 2 an MCF7 Zellen). Überraschenderweise ist dieser Effekt bei den Zellinien DU145, Caco-2, HeLa-MaTu-ADR, und B16F10 besonders ausgeprägt (bis zu 130-fach bessere antiproliferative Aktivität der Beispielverbindungen 1-3 gegenüber der Struktur 692 des Beispiels 1 aus WO 2003/032997). Die Permeabilitätsassays (Tab. 3) zeigen, dass die Beispielverbindungen 1-2 eine gute und freie Permeation über eine geschlossene Caco-2 Zellschicht aufweisen. Die Struktur 692 des Beispiels 1 aus WO 2003/032997 ist durch eine sehr schlechte Permeation in absorptiver Richtung und durch eine hohe Permeation in Efflux-Richtung gekennzeichnet.

Der direkte Vergleich der Beispiele 1-3 mit den analogen 4-N Verbindungen (Vergleichsbeispiele V1-V3) zeigt eine Verbesserung der Kinaseselektivität der Beispielverbindungen 1-3 gegenüber der VEGF-Rezeptorkinase-2 um mindestens den Faktor 2. Die Beispielverbindungen 1-2 zeigen gegenüber den Vergleichsbeispielen eine Verbesserung der Inhibition der CDK4 und zeigen an den Zellinien DU145 und HeLa-MaTu-ADR eine um mehr als den Faktor 2 verstärkte antiproliferative Wirkung.

Diese Daten belegen die Überlegenheit der erfindungsgemäßen Verbindungen (Beispiele 1-3) gegenüber dem nächstliegenden Stand der Technik (WO 2003/032997). Dies wird insbesondere deutlich gezeigt anhand der verstärkten antiproliferativen Aktivität der Beispielverbindungen in den als Chemotherapie-resistent bekannten Zellinien DU145, HeLa-MaTu-ADR und Caco-2.

**Patentansprüche**

1. Verbindungen der allgemeinen Formel (Ia)

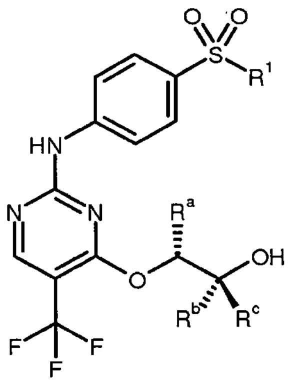

(Ia),

in der

$R^1$ für eine Methylgruppe oder für einen Cyclopropylring steht, und
$R^a$ und $R^b$ für eine Methylgruppe stehen, und
$R^c$ für Wasserstoff oder eine Methylgruppe steht,

sowie deren Salze, Diatereomere und Enantiomere.

**2.** Verbindungen

- (2R,3R)-3-[2-(4-Cyclopropansulfonyl-phenylamino)-5-trifluormethyl-pyrimidin-4-yloxy]-butan-2-ol
- (2R,3R)-3-[2-(4-Methansulfonyl-phenylamino)-5-trifluormethyl-pyrimidin-4-yloxy]-butan-2-ol
- (R)-3-[2-(4-Methansulfonyl-phenylamino)-5-trifluormethyl-pyrimidin-4-yloxy]-2-methylbutan-2-ol

**3.** Verfahren zur Herstellung der Verbindungen gemäß einem der Ansprüche 1 oder 2 umfassend mindestens einer der Schritte

$a_1$) Funktionalisierung der 4-Position von 2,4-Dichlor-5-jod-pyrimidin (1) durch Umsetzung mit einem Alkohol der Formel (2) unter Bildung eines Intermediates der Formel (3),

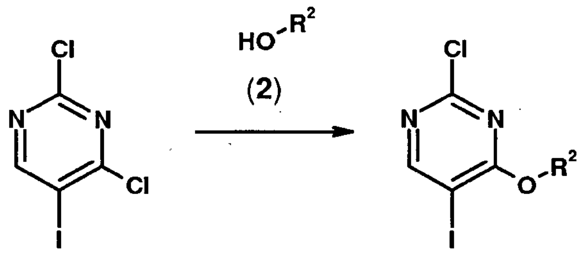

(1) (3)

und nachfolgende Umsetzung von Intermediat (3) unter Bildung des 5-$CF_3$ Intermediates (4)

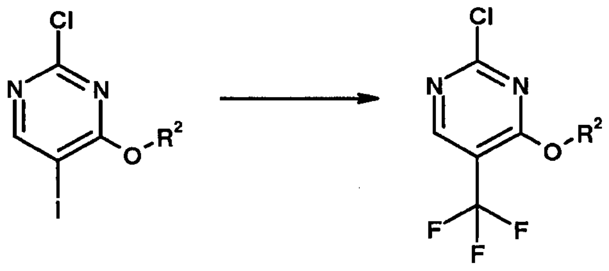

(3) (4)

oder alternativ

$a_2$) direkte Umsetzung von 2,4-Dichlor-5-trifluormethyl-pyrimidin (5) und einem Alkohol der Formel (2) unter Bildung des 5-$CF_3$ Intermediates (4).

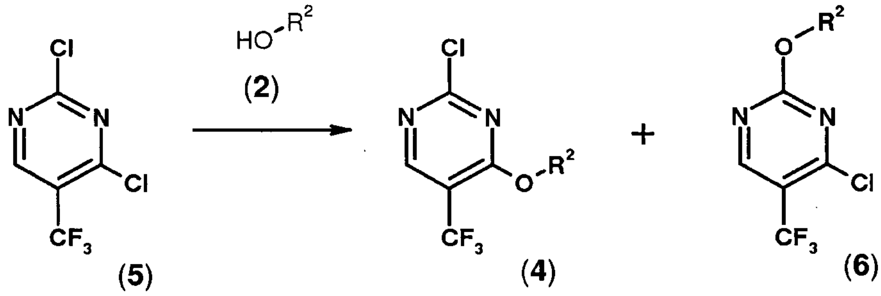

(2)

(5) (4) (6)

b) Oxidation eines Thioethers der Formel (7) zum Sulfon der Formel **(8).**

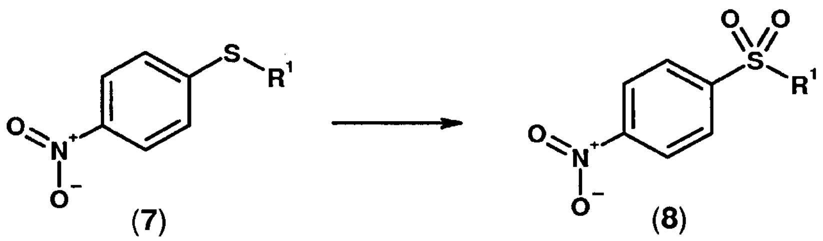

(7) (8)

c) Reduktion der Verbindung der Formel (8) zu einer Verbindung der Formel (9),

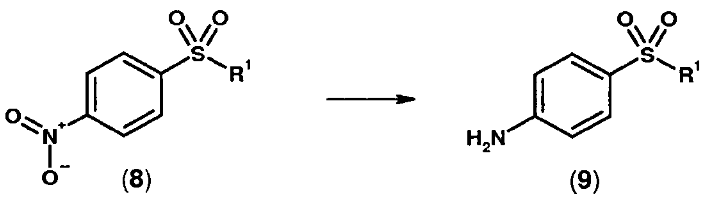

(8) (9)

d) Kupplung der Verbindungen der Formel (4) und (9)

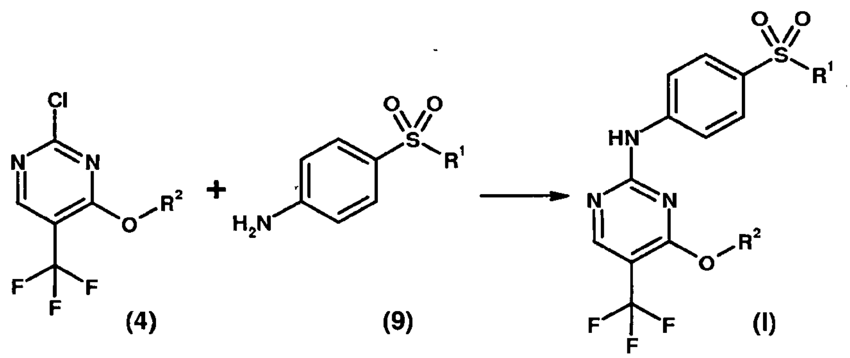

(4) (9) (I)

wobei die Substituenten $R^1$ und $R^2$ die in der allgemeinen Formel (I) gemäß einem der Ansprüche 1 bis 7 angegebenen Bedeutungen aufweisen.

4. Verbindungen gemäß einem der Ansprüche 1 oder 2 zur Verwendung als Arzneimittel.

5. Verwendung einer Verbindung gemäß einem der Ansprüche 1 oder 2 zur Herstellung eines Arzneimittels zur Behandlung von Krebs.

6. Verbindungen gemäß einem der Ansprüche 1 oder 2 zur Verwendung als Arzneimittel gegen Krebs.

7. Pharmazeutische Formulierung enthaltend eine Verbindung gemäß einem der Ansprüche 1 oder 2.

**Claims**

1. Compounds of the general formula (Ia)

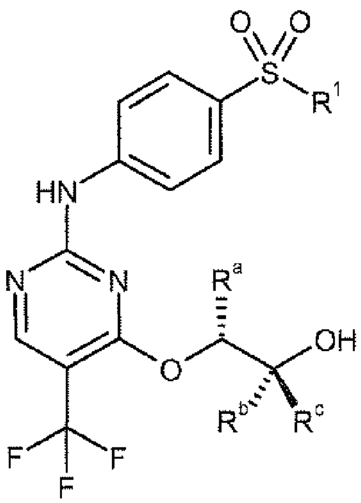

(Ia),

in which

$R^1$ is a methyl group or a cyclopropyl ring, and
$R^a$ and $R^b$ are a methyl group, and
$R^c$ is hydrogen or a methyl group,

and salts, diastereomers and enantiomers thereof.

2. Compounds

- (2R,3R)-3-[2-(4-cyclopropanesulfonylphenylamino)-5-trifluoromethylpyrimidin-4-yloxy]butan-2-ol
- (2R,3R)-3-[2-(4-methanesulfonylphenylamino)-5-trifluoromethylpyrimidin-4-yloxy]butan-2-ol
- (R)-3-[2-(4-mmethanesulfonylphenylamino)-5-trifluoromethyl-pyrimidin-4-yloxy]-2-methylbutan-2-ol

**3.** Process for the preparation of the compounds according to one of Claims 1 and 2, comprising at least one of the steps

$a_1$) functionalization of the 4-position of 2,4-dichloro-5-iodopyrimidine **(1)** by reaction with an alcohol of the formula **(2)** to form an intermediate of the formula **(3),**

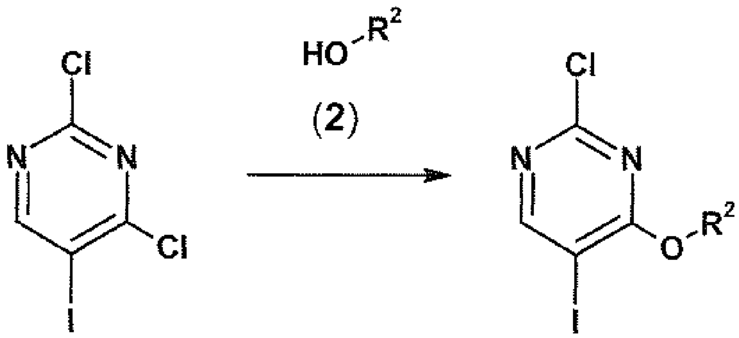


(1) (3)

and subsequent reaction of intermediate **(3)** to form the 5-$CF_3$ intermediate **(4)**

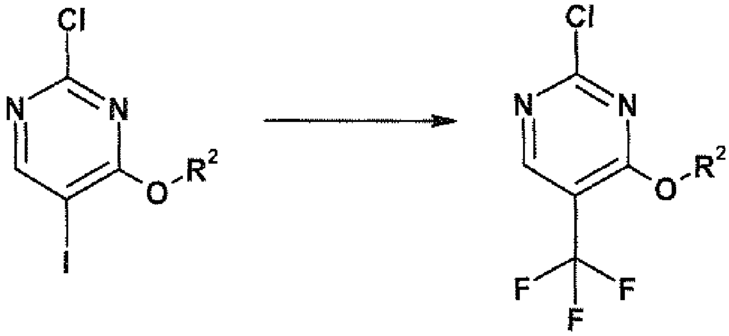

(3) (4)

or alternatively

$a_2$) direct reaction of 2,4-dichloro-5-trimethylpyrimidine **(5)** and an alcohol of the formula **(2)** to form the 5-$CF_3$ intermediate **(4),**

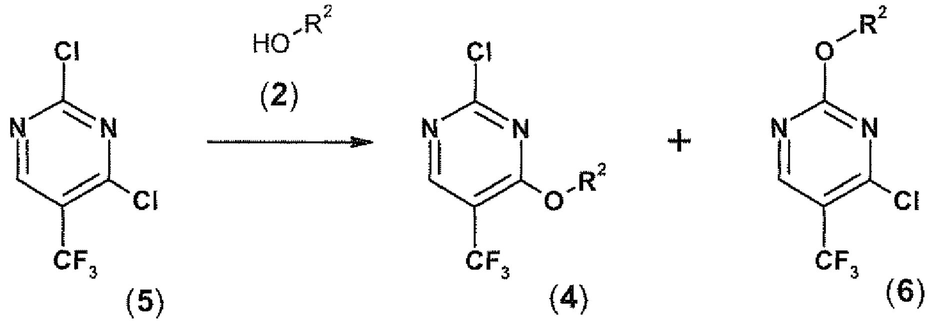
(5) (4) (6)

b) oxidation of a thioether of the formula **(7)** to give the sulphone of the formula **(8),**

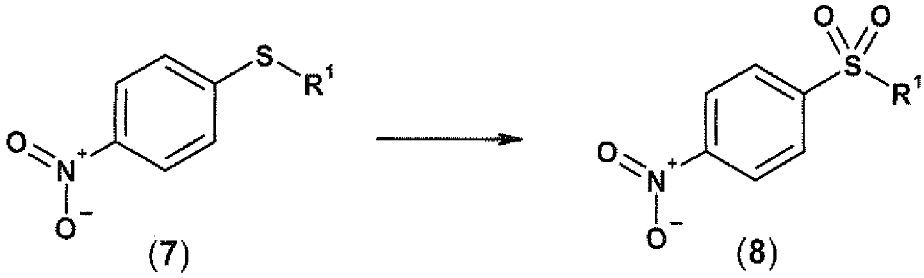
(7) (8)

c) reduction of the compound of the formula **(8)** to a compound of the formula **(9),**

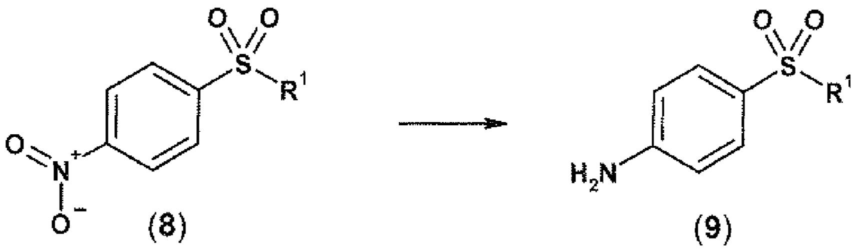
(8) (9)

d) coupling of the compounds of the formula **(4)** and **(9)**

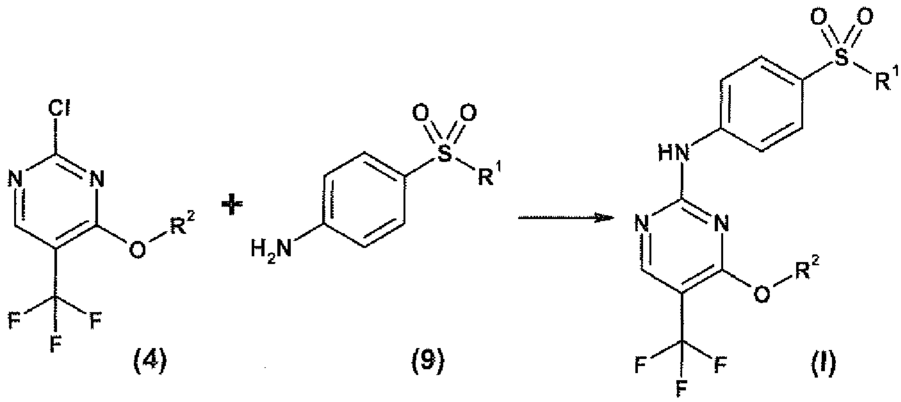
(4) (9) (I)

where the substituents $R^1$ and $R^2$ have the meanings given in the general formula (I) according to one of Claims 1 and 2.

**4.** Compounds according to one of Claims 1 and 2 for use as medicament.

**5.** Use of a compound according to one of Claims 1 and 2 for producing a medicament for the treatment of cancer.

**6.** Compounds according to one of Claims 1 and 2 for use as medicament against cancer.

**7.** Pharmaceutical formulation comprising a compound according to one of Claims 1 and 2.

**Revendications**

**1.** Composés de formule générale (Ia)

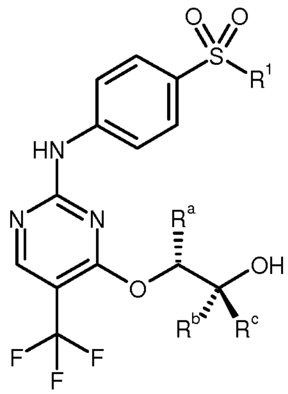

(Ia),

dans laquelle

$R^1$ représente un groupe méthyle ou un cycle cyclopropyle, et
$R^a$ et $R^b$ représentent un groupe méthyle, et
$R^c$ représente un atome d'hydrogène ou un groupe méthyle,

ainsi que leurs sels, diastéréoisomères et énantiomères.

**2.** Composés

- (2R,3R)-3-[2-(4-cyclopropanesulfonyl-phénylamino)-5-trifluorométhyl-pyrimidin-4-yloxy]-butan-2-ol
- (2R,3R)-3-[2-(4-méthanesulfonyl-phénylamino)-5-trifluorométhyl-pyrimidin-4-yloxy]-butan-2-ol
- (R)-3-[2-(4-méthanesulfonyl-phénylamino)-5-trifluorométhyl-pyrimidin-4-yloxy]-2-méthyl-butan-2-ol.

**3.** Procédé pour la préparation des composés selon l'une quelconque des revendications 1 et 2, comprenant au moins l'une des étapes

$a_1$) fonctionnalisation de la position 4 de la 2,4-dichloro-5-iodo-pyrimidine **(1)** par mise en réaction avec un alcool de formule **(2),** avec formation d'un composé intermédiaire de formule **(3),**

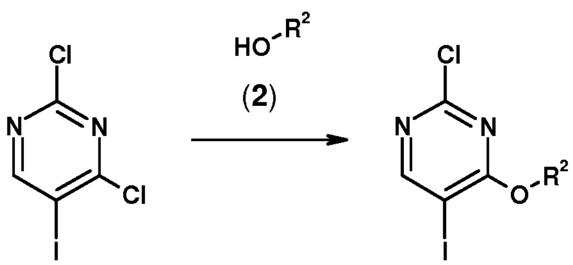

(**1**) (**3**)

et réaction subséquente du composé intermédiaire **(3)**, avec formation du composé intermédiaire 5-$CF_3$ **(4)**

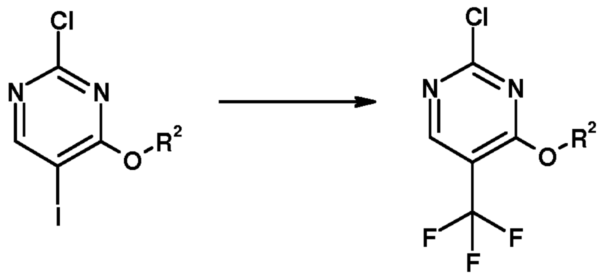

(**3**) (**4**)

ou bien

$a_2$) réaction directe de la 2,4-dichloro-5-trifluoro-méthyl-pyrimidine **(5)** et d'un alcool de formule **(2)** avec formation du composé intermédiaire 5-$CF_3$ **(4)**.

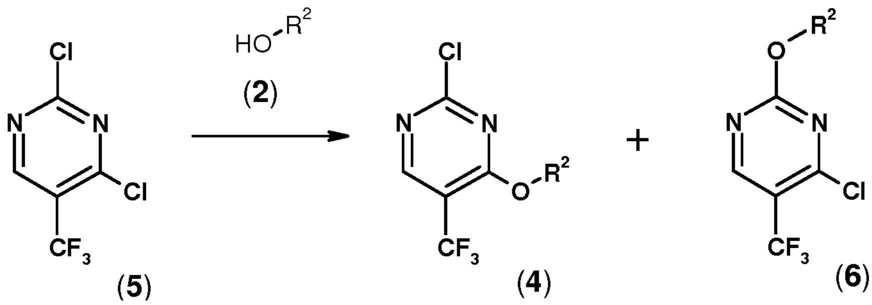

(**2**)

(**5**) (**4**) + (**6**)

b) oxydation d'un thioéther de formule **(7)** conduisant à la sulfone de formule **(8)**.

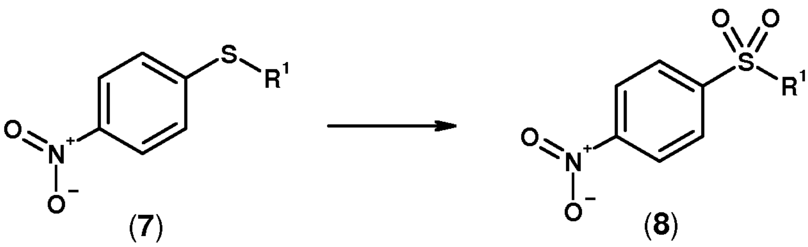

(**7**) (**8**)

c) réduction du composé de formule **(8)** en un composé de formule **(9)**,

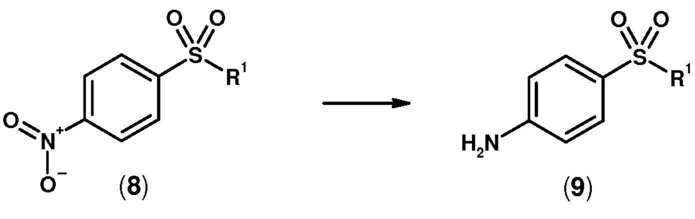

(**8**) (**9**)

d) couplage des composés de formules **(4)** et **(9)**

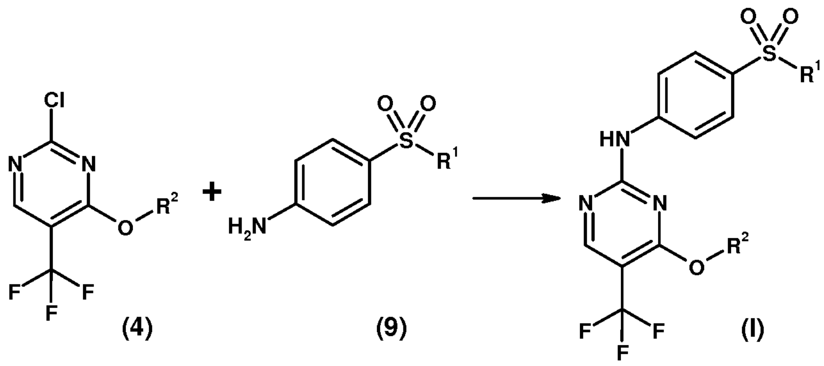

(4) (9) (I)

Les substituants $R^1$ et $R^2$ ayant les significations indiquées dans la formule générale (I) selon l'une quelconque des revendications 1 à 7.

**4.** Composés selon l'une quelconque des revendications 1 et 2 pour utilisation en tant que médicament.

**5.** Utilisation d'un composé selon l'une quelconque des revendications 1 et 2, pour la fabrication d'un médicament destiné au traitement du cancer.

**6.** Composés selon l'une quelconque des revendications 1 et 2 pour utilisation en tant que médicament contre le cancer.

**7.** Composition pharmaceutique contenant un composé selon l'une quelconque des revendications 1 et 2.

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- DE 4029650
- WO 9919305 A
- WO 0114375 A
- WO 9902162 A
- WO 0204429 A
- WO 0012486 A
- WO 0039101 A
- WO 02096888 A
- WO 03076437 A
- WO 2005037800 A
- WO 2003032997 A
- WO 2007071455 A, U. Lücking
- WO 2006081388 A, T.M. Caldwell
- WO 2004048343 A, J. Bryant


### In der Beschreibung aufgeführte Nicht-Patentliteratur


- Remington's Pharmaceutical Science. Mack Publishing Company, 1980
- **P.J. KOCIENSKI.** Protecting Groups. Georg Thieme Verlag, 1994
- **G.E. CARR ; R.D. CHAMBERS ; T.F. HOLMES.** *J. Chem. Soc. Perkin Trans.,* 1988, vol. 1, 921
- **F. COTTET ; M. SCHLOSSER.** *Eur. J. Org. Chem.,* 2002, 327
- **F.G. NJOROGE et al.** *J. Med. Chem,* 1997, vol. 40, 4290
- **R.C. LAROCK.** Comprehensive Organic Transformations. VCH, 1989, 411
- **E. BLOCK et al.** *J. Am. Chem. Soc.,* 1992, vol. 114, 3492